# EUROPEAN PATENT APPLICATION

(11) **EP 4 613 285 A1**
(43) Date of publication of application: **10.09.2025**
(21) Application number: 23885140.6
(22) Date of filing: 06.11.2023
(51) Int. Cl.: A61K 38/48, A61K 38/49, A61P 25/00, A61P 25/16, A61P 25/28

(54) **METHOD AND DRUG FOR PROMOTING REMOVAL OF PATHOLOGICAL PROTEINS BY UBIQUITIN PROTEASOME SYSTEM AND AUTOPHAGY LYSOSOME SYSTEM**

(30) Priority: 04.11.2022 WO PCT/CN2022/129818
(71) Applicant: Talengen International Limited, Hong Kong 999077 (HK)
(72) Inventor: LI, Jinan, Beijing 100089 (CN)
(74) Representative: CMS Cameron McKenna Nabarro Olswang LLP
(86) International application number: PCT/CN2023/130018
(87) International publication number: WO 2024/094216

(57) **Abstract**

A method for regulating and optimizing a ubiquitin proteasome system and/or an autophagy lysosome system in order to promote the degradation of intracellular and extracellular pathological proteins; a drug for promoting the degradation of intracellular and extracellular pathological proteins; and the use thereof.

## Description

### TECHNICAL FIELD

The present application relates to use of plasminogen activation pathway-related compounds such as plasminogen or plasmin for promoting the removal of pathological proteins by a ubiquitin proteasome system and an autophagy lysosome system.

### BACKGROUND

Chronic neurodegenerative diseases are a disease state of neuronal loss in the brain and spinal cord cells, including Alzheimer's disease, Huntington's disease, Parkinson's disease, amyotrophic lateral sclerosis, spinal muscular atrophy, etc. Studies have found that it is lesions caused by ubiquitin-dependent processes, including ubiquitination degradation of the 26S proteasome and abnormal autophagy through the lysosomal pathway, which have serious effects on neural development, homeostasis and the occurrence of diseases. Therefore, ubiquitination degradation and autophagy are essential for neural activity and are involved in synaptogenesis and cell-cell interactions. In the adult central and peripheral nervous systems, based on non-dividing cells, the ubiquitination and deubiquitination of proteins are crucial for the survival of neurons. Most chronic neurodegenerative diseases in the elderly are attributed to the accumulation of total protein, and often exist as nuclear contents.

The ubiquitin proteasome system (UPS) and the autophagy lysosome system are intracellular protein degradation systems, and their interaction can promote the degradation of pathological proteins. People hope to find substances that can promote the interaction between the ubiquitin proteasome system (UPS) and the autophagy lysosome system, thereby promoting the removal of pathological proteins that cause disease in cells.

### SUMMARY

The present application has found that plasminogen can promote the interaction between the ubiquitin proteasome system (UPS) and the autophagy lysosome system to some extent, and promote the removal of pathological proteins inside and outside the cell. Specifically, the present application relates to the following items:
1. A method for promoting removal of a pathological protein by a ubiquitin proteasome system and an autophagy lysosome system, comprising administering to a subject an effective amount of one or more compounds selected from the group consisting of : a component of a plasminogen activation pathway, a compound capable of activating plasminogen directly or activating plasminogen indirectly by activating an upstream component of a plasminogen activation pathway, a compound mimicking the activity of plasminogen or plasmin, a compound capable of up-regulating the expression of plasminogen or a plasminogen activator, a plasminogen analog, a plasmin analog, a tPA or uPA analog, and an antagonist of a fibrinolysis inhibitor.
2. The method according to item 1, wherein the component of the plasminogen activation pathway is selected from the group consisting of plasminogen, recombinant human plasmin, Lys-plasminogen, Glu-plasminogen, plasmin, plasminogen and plasmin variants and analogs containing one or more kringle domains and protease domains of plasminogen and plasmin, mini-plasminogen, mini-plasmin, micro-plasminogen, micro-plasmin, delta-plasminogen, delta-plasmin, a plasminogen activator, tPA, and uPA.
3. The method according to item 1, wherein the antagonist of the fibrinolytic inhibitor is an inhibitor of PAI-1, a complement C1 inhibitor, α2 antiplasmin or α2 macroglobulin, such as an antibody.
4. The method according to any one of items 1 to 3, wherein the compound has one or more of the following activities: removing different types of pathological proteins outside the cell directly and/or entering the cell and/or the nucleus directly, promoting the removal of pathological proteins by a ubiquitin proteasome system, promoting the removal of pathological proteins by an autophagy lysosome system, regulating to optimize the expression and/or activity of members of a ubiquitin system, regulating to optimize the expression and/or activity of LC3, regulating to optimize the expression and/or activity of members of an autophagy lysosome system, and regulating to optimize (especially promoting) the expression of LAMP2.
5. The method according to any one of items 1 to 4, wherein the compound is plasminogen or plasmin.
6. The method according to any one of items 1 to 5, wherein the plasminogen is Glu-plasminogen, Lys-plasminogen, or a conservatively substituted variant thereof.
7. The method according to any one of items 1 to 6, wherein the plasminogen has at least 75%, 80%, 85%, 90%, 95%, 96%, 97%, 98% or 99% sequence identity with SEQ ID NO: 2 and has lysine binding activity and/or proteolytic activity of plasminogen.
8. The method according to any one of items 1 to 7, wherein the plasminogen comprises one or more selected from the group consisting of:
   1) a serine protease domain having the amino acid sequence represented by SEQ ID NO: 14;
   2) a serine protease domain having at least 80%, 90%, 95%, 96%, 97%, 98%, or 99% identity with SEQ ID NO: 14 and retaining proteolytic activity;
   3) a Kringle domain being one or more selected from the group consisting of Kringle 1, Kringle 2, Kringle 3, Kringle 4 and Kringle 5; and
   4) a Kringle domain having at least 80%, 90%, 95%, 96%, 97%, 98%, or 99% identity with one or more selected from the group consisting of Kringle 1, Kringle 2, Kringle 3, Kringle 4 and Kringle 5, and retaining lysine binding activity.
9. The method according to any one of items 1 to 8, wherein the plasminogen is selected from Glu-plasminogen, Lys-plasminogen, mini-plasminogen, micro-plasminogen, delta-plasminogen or a variant thereof retaining the proteolytic activity of plasminogen.
10. The method according to any one of items 1 to 9, wherein the plasminogen comprises the amino acid sequence represented by SEQ ID NO: 2, 6, 8, 10, or 12, or comprises a conservatively substituted variant of the amino acid sequence represented by SEQ ID NO: 2, 6, 8, 10, or 12.
11. The method according to any one of items 1-10, wherein the plasminogen is used in combination with one or more other treatment methods or drugs.
12. The method according to item 11, wherein the other treatment method comprises cell therapy (comprising stem cell therapy), supportive therapy, and physical therapy.
13. The method according to any one of items 1 to 12, wherein the plasminogen is administered by nasal inhalation, nebulized inhalation, nasal drops, eye drops, ear drops, intravenous, intraperitoneal, subcutaneous, sublingual, intracranial, intrathecal, intra-arterial (e.g., via carotid artery), or intramuscular administration.

On the other hand, the present application also relates to:
1. A method for preventing or treating a disease caused by pathological protein aggregation or ubiquitin/lysosome dysfunction, comprising administering to a subject an effective amount of one or more compounds selected from the group consisting of: a component of a plasminogen activation pathway, a compound capable of activating plasminogen directly or activating plasminogen indirectly by activating an upstream component of a plasminogen activation pathway, a compound mimicking the activity of plasminogen or plasmin, a compound capable of up-regulating the expression of plasminogen or a plasminogen activator, a plasminogen analog, a plasmin analog, a tPA or uPA analog, and an antagonist of a fibrinolysis inhibitor.
2. The method according to item 1, wherein the component of the plasminogen activation pathway is selected from the group consisting of plasminogen, recombinant human plasmin, Lys-plasminogen, Glu-plasminogen, plasmin, plasminogen and plasmin variants and analogs containing one or more kringle domains and protease domains of plasminogen and plasmin, mini-plasminogen, mini-plasmin, micro-plasminogen, micro-plasmin, delta-plasminogen, delta-plasmin, a plasminogen activator, tPA, and uPA.
3. The method according to item 1, wherein the antagonist of the fibrinolytic inhibitor is an inhibitor of PAI-1, a complement C1 inhibitor, α2 antiplasmin or α2 macroglobulin, such as an antibody.
4. The method according to any one of items 1 to 3, wherein the compound has one or more of the following activities: removing different types of pathological proteins outside the cell directly and/or entering the cell and/or the nucleus directly, promoting the removal of pathological proteins by a ubiquitin proteasome system, promoting the removal of pathological proteins by an autophagy lysosome system, regulating to optimize the expression and/or activity of members of a ubiquitin system, regulating to optimize the expression and/or activity of LC3, regulating to optimize the expression and/or activity of members of an autophagy lysosome system, and regulating to optimize (especially promoting) the expression of LAMP2.
5. The method according to any one of items 1 to 4, wherein the compound is plasminogen or plasmin.
6. The method according to any one of items 1 to 5, wherein the plasminogen is Glu-plasminogen, Lys-plasminogen, or a conservatively substituted variant thereof.
7. The method according to any one of items 1 to 6, wherein the plasminogen has at least 75%, 80%, 85%, 90%, 95%, 96%, 97%, 98% or 99% sequence identity with SEQ ID NO: 2 and has lysine binding activity and/or proteolytic activity of plasminogen.
8. The method according to any one of items 1 to 7, wherein the plasminogen comprises one or more selected from the group consisting of:
   1) a serine protease domain having the amino acid sequence represented by SEQ ID NO: 14;
   2) a serine protease domain having at least 80%, 90%, 95%, 96%, 97%, 98%, or 99% identity with SEQ ID NO: 14 and retaining proteolytic activity;
   3) a Kringle domain being one or more selected from the group consisting of Kringle 1, Kringle 2, Kringle 3, Kringle 4 and Kringle 5; and
   4) a Kringle domain having at least 80%, 90%, 95%, 96%, 97%, 98%, or 99% identity with one or more selected from the group consisting of Kringle 1, Kringle 2, Kringle 3, Kringle 4 and Kringle 5, and retaining lysine binding activity.
9. The method according to any one of items 1 to 8, wherein the plasminogen is selected from the group consisting of Glu-plasminogen, Lys-plasminogen, mini-plasminogen, micro-plasminogen, delta-plasminogen or a variant thereof retaining the proteolytic activity of plasminogen.
10. The method according to any one of items 1 to 9, wherein the plasminogen comprises the amino acid sequence represented by SEQ ID NO: 2, 6, 8, 10, or 12, or comprises a conservatively substituted variant of the amino acid sequence represented by SEQ ID NO: 2, 6, 8, 10, or 12.
11. The method according to any one of items 1 to 10, wherein the plasminogen is used in combination with one or more other treatment methods or drugs.
12. The method according to claim 11, wherein the other treatment method comprises cell therapy (comprising stem cell therapy), supportive therapy, and physical therapy.
13. The method according to any one of items 1 to 12, wherein the plasminogen is administered by nasal inhalation, nebulized inhalation, nasal drops, eye drops, ear drops, intravenous, intraperitoneal, subcutaneous, sublingual, intracranial, intrathecal, intra-arterial (e.g., via carotid artery), or intramuscular administration. In some embodiments, the method according to item 12 or 11, wherein the other treatment method comprises cell therapy (including stem cell therapy), gene therapy, supportive therapy and physical therapy.
14. The method according to any one of items 1 to 12, wherein the plasminogen is administered by nasal inhalation, nebulized inhalation, nasal drops, eye drops, ear drops, intravenous, intraperitoneal, subcutaneous, sublingual, intracranial, intrathecal, intra-arterial (e.g., via carotid artery), or intramuscular administration.

In some embodiments, the above disease caused by pathological protein aggregation includes: Alzheimer's disease, Parkinson's disease, amyotrophic lateral sclerosis, prion-like disease (Creutzfeldt-Jakob disease), Gerstmann syndrome, dispersive or familial fatal insomnia and kuru disease, Huntington's disease, spinocerebellar ataxia type 3, dentatorubral-pallidoluysian atrophy, diabetes, spongiform encephalopathy, macular degeneration, atherosclerosis, familial British dementia, familial Danish dementia, Down's syndrome, hemodialysis related amyloid deposition disease, amyloid cardiomyopathy, systemic amyloidosis, corneal dystrophy, glomerulonephritis, and amyloid body.

In some embodiments, the above disease caused by ubiquitin/lysosome dysfunction includes: Fanconi anemia, Xeroderma pigmentosum, Cockayne syndrome, cancer, Cowden syndrome, Parkinson's disease, genomic instability, metabolic syndrome, myatrophy, Von Hippel Lindau syndrome, multiple myeloma, hereditary pseudo hyperaldosteronism (RIDDLE syndrome), Huntington's disease, X-linked lymphoproliferative disease, Crohn's disease, Alzheimer's disease, breast cancer and ovarian cancer, muscular amylopectinosis, amyotrophic lateral sclerosis, spinal muscular atrophy, systemic lupus erythematosus, childhood ataxia, X-linked parkinsonism syndrome with spasticity, multisystem disorder, diabetes, multiple sclerosis, cystinosis, Vici syndrome, Gaucher's disease, frontotemporal dementia (heterozygous) or neuronal ceroid lipofuscinosis (homozygous), Danon's cardiomyopathy, cortical atrophy, epilepsy, autosomal recessive spinal and cerebellar ataxia, hereditary spastic paraplegia, beta-propeller protein-associated neurodegeneration (BPAN), autism spectrum disorder, frontotemporal dementia (FTD), inflammatory bowel disease, nonalcoholic fatty liver disease, pulmonary tuberculosis, Rett syndrome, osteopetrosis, Charcot-Marie-Tooth type 2B disease, juvenile arthritis, Snyder-Robinson syndrome (SRS), Wiskott-Aldrich syndrome, primary microcephaly, hereditary sensory and autonomic neuropathy type II, primary open angle glaucoma (POAG), Paget's disease of the bone (PGD), colon cancer, lung cancer, brain cancer, autosomal recessive and sporadic early-onset Parkinson's disease, Zellweger syndrome spectrum disorders, distal myopathy, ulcerative colitis, familial Mediterranean fever, ataxia with spasticity, Fabry disease, Gaucher disease, lysosomal acid lipase deficiency, mucopolysaccharidosis, and Angelman syndrome.

In some particular embodiments, the plasminogen pathway activator is administered in combination with one or more other drugs or therapies, preferably, the therapy comprises cell therapy (e.g., stem cell therapy) and gene therapy, e.g., antisense RNA, small molecule splicing modifiers.

In some particular embodiments, the plasminogen pathway activator is a component of the plasminogen activation pathway, e.g., plasminogen. In some particular embodiments, the plasminogen comprises or has an amino acid sequence having at least 75%, 80%, 85%, 90%, 95%, 96%, 97%, 98% or 99% sequence identity with the amino acid sequence represented by SEQ ID NO: 2, 6, 8, 10 or 12, and has plasminogen activity and/or lysine binding activity. In some embodiments, the plasminogen is a protein with an addition, deletion, and/or substitution of 1-100, 1-90, 1-80, 1-70, 1-60, 1-50, 1-45, 1-40, 1-35, 1-30, 1-25, 1-20, 1-15, 1-10, 1-5, 1-4, 1-3, 1-2, or 1 amino acid(s) based on SEQ ID NO: 2, 6, 8, 10 or 12, and having plasminogen activity and/or lysine binding activity. In some particular embodiments, the plasminogen activity is the proteolytic activity of plasminogen. In some particular embodiments, the plasminogen is a protein comprising an active fragment of plasminogen and having plasminogen activity and/or lysine binding activity. In some particular embodiments, the plasminogen activity is the proteolytic activity of plasminogen. In some particular embodiments, the active fragment of plasminogen comprises or has a serine protease domain of plasminogen or a plasminogen protease domain. In some particular embodiments, the amino acid sequence of the active fragment of plasminogen is represented by SEQ ID NO: 14. In some particular embodiments, the plasminogen is selected from the group consisting of: Glu-plasminogen (a human full-length plasminogen), Lys-plasminogen (a human full-length plasminogen after cleavage between amino acid positions 76-77), mini-plasminogen (comprising Kringle 5 (K5) and a serine protease domain), micro-plasminogen (comprising a serine protease domain), delta-plasminogen (comprising Kringle 1 and a serine protease domain), or a variant thereof retaining plasminogen activity. In some particular embodiments, the plasminogen is a human full-length plasminogen, or a variant or fragment thereof that still retains plasminogen activity and/or lysine binding activity. In some embodiments, the plasminogen is a human plasminogen orthologue from a primate or a rodent, or a variant or fragment thereof still retaining plasminogen activity and/or lysine binding activity. In some embodiments, the plasminogen comprises an amino acid sequence represented by SEQ ID NO: 2, 6, 8, 10 or 12. In some embodiments, the plasminogen is human natural plasminogen.

In some particular embodiments, the plasminogen pathway activator is administered systemically or locally, e.g., administered by intravenous, intramuscular, nasal inhalation, aerosol inhalation, or nasal drop. In some embodiments, the subject is a human. In some embodiments, the subject is deficient in or lack of plasminogen. In some embodiments, the deficiency or lack is congenital, secondary and/or local. In some embodiments, the plasminogen is administrated daily, or every second day or every third day consecutively at a dose of 0.0001-2000 mg/kg, 0.001-800 mg/kg, 0.01-600 mg/kg, 0.1-400 mg/kg, 1-200 mg/kg, 1-100 mg/kg, 10-100mg/kg (calculating by per kilogram of body weight); or 0.0001-2000 mg/cm², 0.001-800 mg/cm², 0.01-600 mg/cm², 0.1-400 mg/cm², 1-200 mg/cm², 1-100 mg/cm², 10-100 mg/cm² (calculating by per square centimeter of body surface area).

In one aspect, the present application also relates to a pharmaceutical composition, medicament, preparation, kit, and product used in the above method, which comprises the above plasminogen pathway activator, e.g., the above plasminogen.

In some embodiments, the pharmaceutical composition, medicament, preparation comprises a pharmaceutically acceptable carrier and a plasminogen pathway activator, for example, a component of the plasminogen activation pathway, e.g., plasminogen. In some embodiments, the kit and product comprises one or more containers containing the pharmaceutical composition, medicament or preparation therein. In some embodiments, the kit or product further comprises a label or instructions for use indicating that the plasminogen pathway activator, for example, a component of the plasminogen activation pathway, e.g., plasminogen is used in the above method. In some embodiments, the kit or product further comprises one or more additional containers containing one or more additional drugs.

In one aspect, the present application also relates to a plasminogen pathway activator, such as plasminogen as described above, for the use as described above.

In one aspect, the present application also relates to use of the therapeutically effective amount of the above plasminogen pathway activator in the preparation of a pharmaceutical composition, medicament, preparation, kit, and product for the above methods.

In some embodiments, the plasminogen pathway activator is one or more selected from the group consisting of: a component of plasminogen activation pathway, a compound capable of activating plasminogen directly or activating plasminogen indirectly by activating an upstream component of plasminogen activation pathway, a compound mimicking the activity of plasminogen or plasmin, a compound capable of up-regulating the expression of plasminogen or an activator of plasminogen, an analog of plasminogen, an analog of plasmin, an analog of tPA or uPA, and an antagonist of a fibrinolysis inhibitor.

In some particular embodiments, the component of plasminogen activation pathway is selected from the group consisting of: plasminogen, recombinant human plasmin, Lys-plasminogen, Glu-plasminogen, plasmin, a variant and analog of plasminogen and plasmin comprising one or more kringle domains and/or protease domains of plasminogen and plasmin, mini-plasminogen, mini-plasmin, micro-plasminogen, micro-plasmin, delta-plasminogen, delta-plasmin, an activator of plasminogen, tPA and uPA. In some particular embodiments, the antagonist of the fibrinolysis inhibitor is an antagonist of PAI-1, a complement C1 inhibitor, α2 antiplasmin or α2 macroglobulin, e.g., an antibody of PAI-1, a complement C1 inhibitor, α2 antiplasmin or α2 macroglobulin.

In some particular embodiments, the plasminogen pathway activator is administered in combination with one or more other drugs and/or therapies, preferably, the therapy comprises cell therapy (e.g., stem cell therapy) and gene therapy, such as antisense RNA, small molecule splicing modifiers.

In some particular embodiments, the plasminogen pathway activator is a component of the plasminogen activation pathway, e.g., plasminogen. In some particular embodiments, the plasminogen comprises or has an amino acid sequence having at least 75%, 80%, 85%, 90%, 95%, 96%, 97%, 98% or 99% sequence identity with the amino acid sequence represented by SEQ ID NO: 2, 6, 8, 10 or 12, and has the plasminogen activity and/or lysine binding activity. In some embodiments, the plasminogen is a protein with an addition, deletion, and/or substitution of 1-100, 1-90, 1-80, 1-70, 1-60, 1-50, 1-45, 1-40, 1-35, 1-30, 1-25, 1-20, 1-15, 1-10, 1-5, 1-4, 1-3, 1-2, or 1 amino acid(s) based on SEQ ID NO: 2, 6, 8, 10 or 12, and having plasminogen activity and/or lysine binding activity. In some particular embodiments, the plasminogen activity is the proteolytic activity of plasminogen. In some particular embodiments, the plasminogen is a protein comprising an active fragment of plasminogen and having plasminogen activity and/or lysine binding activity. In some particular embodiments, the plasminogen activity is the proteolytic activity of plasminogen. In some particular embodiments, the active fragment of plasminogen comprises or has a serine protease domain of plasminogen or a plasminogen protease domain. In some particular embodiments, the amino acid sequence of the active fragment of plasminogen is represented by SEQ ID NO: 14. In some particular embodiments, the plasminogen is selected from the group consisting of: Glu-plasminogen (a human full-length plasminogen), Lys-plasminogen (a human full-length plasminogen after cleavage between amino acid positions 76-77), mini-plasminogen (comprising Kringle 5 (K5) and a serine protease domain), micro-plasminogen (comprising a serine protease domain), delta-plasminogen (comprising Kringle 1 and a serine protease domain), or a variant thereof retaining plasminogen activity. In some particular embodiments, the plasminogen is a human full-length plasminogen, or a variant or fragment thereof that still retains plasminogen activity and/or lysine binding activity. In some embodiments, the plasminogen is a human plasminogen orthologue from a primate or a rodent, or a variant or fragment thereof still retaining plasminogen activity and/or lysine binding activity. In some embodiments, the plasminogen comprises an amino acid sequence represented by SEQ ID NO: 2, 6, 8, 10 or 12. In some embodiments, the plasminogen is human natural plasminogen.

In some embodiments, the plasminogen pathway activator, for example, a component of the plasminogen activation pathway, e.g., plasminogen, is administered in combination with one or more other drugs and/or therapies. In some embodiments, the plasminogen pathway activator, for example, a component of the plasminogen activation pathway, e.g. plasminogen is administered intravenously, intramuscularly, intrathecally, by nasal inhalation, aerosol inhalation, by nasal drop or eye drop.

In some embodiments, the pharmaceutical composition, medicament, preparation comprises a pharmaceutically acceptable carrier and a plasminogen pathway activator, for example, a component of the plasminogen activation pathway, e.g., plasminogen. In some embodiments, the kit and product comprises one or more containers containing the pharmaceutical composition, medicament or preparation therein. In some embodiments, the kit or product further comprises a label or instructions for use indicating that the plasminogen pathway activator, for example, a component of the plasminogen activation pathway, e.g., plasminogen is used in the above use.

In some embodiments, the kit or product further comprises one or more additional containers containing one or more additional drugs.

The present application explicitly encompasses all the combinations of the technical features belonging to the embodiments of the present application, and these combined technical solutions have been explicitly disclosed in this application, just as the separately and explicitly disclosed above technical solutions. In addition, the present application also explicitly encompasses the combinations of each embodiment and its elements, and the combined technical solutions are explicitly disclosed herein.

### BRIEF DESCRIPTION OF THE DRAWINGS

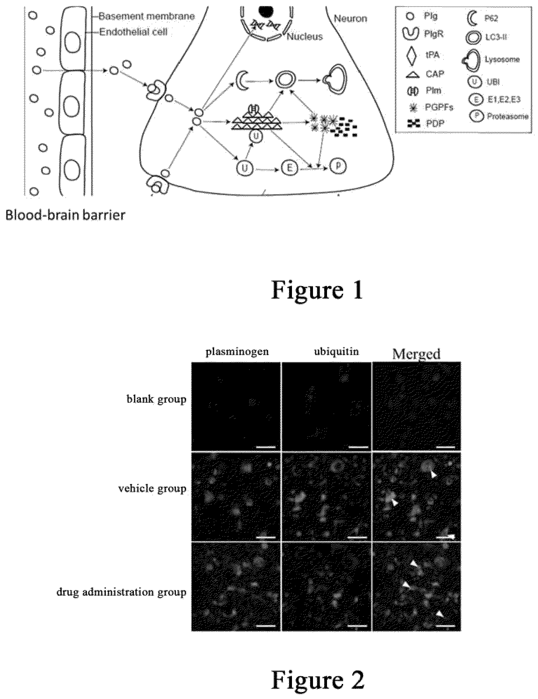

Figure 1 shows schematic diagram of the interaction between plasminogen and the ubiquitin proteasome system and the autophagy lysosome system to promote the degradation of pathological proteins in the central nervous system. Blood-brain barrier, basement membrane, endothelial cells, plasminogen (Plg), plasminogen receptor (PlgR), tissue-type plasminogen activator (tPA), conformationally abnormal proteins (CAP), plasmin (Plm), plasmin generated protein fragments (PGPFs), plasmin degradation products (PDP), lysosome, ubiquitin (UBI), ubiquitin activating enzyme (E1), ubiquitin conjugating enzyme (E2), ubiquitin ligase (E3), proteasome, and nucleus. Existing results show that plasminogen can enter cells and interact with the intracellular protein degradation system - the ubiquitin proteasome system (UPS) and the autophagy lysosome system. Plasminogen can thus promote the degradation of abnormally deposited central nervous system pathological proteins in cells and improve central nervous system degenerative diseases.
Figure 2 shows the localization of plasminogen and the co-localization of plasminogen and ubiquitin in the spinal cord tissue of the amyotrophic lateral sclerosis model mice. The results show that the positive staining of plasminogen (green fluorescence) in the spinal cord tissue in the drug administration group is significantly more than that in the vehicle group, indicating that the plasminogen can enter the spinal cord tissue and be enriched in the spinal cord tissue. In addition, plasminogen is present in the cytoplasm and nucleus. Plasminogen and ubiquitin (red fluorescence) co-localize in the cytoplasm (as shown by Δ) (Figure 2), and the ubiquitin expression level in the spinal cord tissue in the drug administration group seems to be less than that in the vehicle group. This shows that plasminogen can enter the spinal cord tissue, enter the cell, co-localize with ubiquitin, and reduce the expression of ubiquitin in the amyotrophic lateral sclerosis model mice. This suggests that plasminogen may interact with ubiquitin.

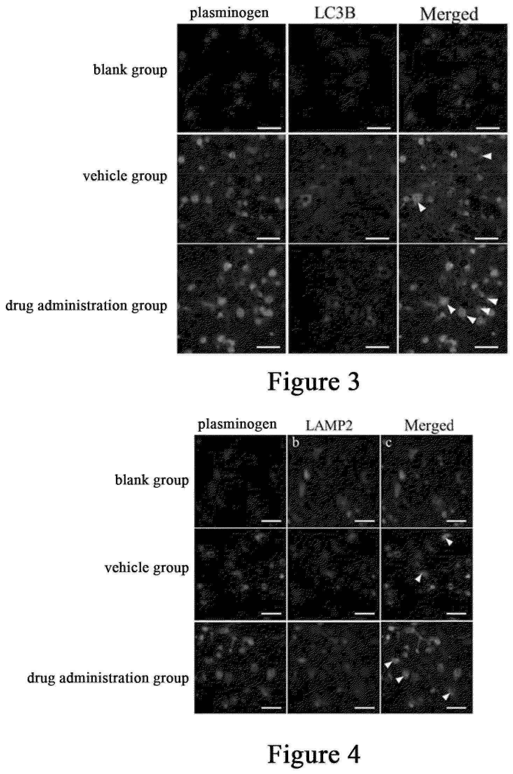

Figure 3 shows the localization of plasminogen and the co-localization of plasminogen and LC3B in the spinal cord tissue of the amyotrophic lateral sclerosis model mice. The results show that the positive staining of plasminogen (green fluorescence) in the spinal cord tissue in the drug administration group is significantly more than that in the vehicle group, indicating that the plasminogen can enter the spinal cord tissue and be enriched in the spinal cord tissue. In addition, plasminogen is present in the cytoplasm and nucleus. Plasminogen and LC3B (red fluorescence) co-localize in the cytoplasm (as shown by Δ), and the expression level of LC3B in the spinal cord tissue in the drug administration group seems to be less than that in the vehicle group. This shows that in amyotrophic lateral sclerosis model mice, plasminogen can enter the spinal cord tissue, enter the cell, and co-localize with LC3B, suggesting that there is an interaction between plasminogen and LC3B.
Figure 4 shows the localization of plasminogen and the co-localization of plasminogen and LAMP2 in the spinal cord tissue of the amyotrophic lateral sclerosis model mice. The results show that the positive staining of plasminogen (green fluorescence) in the spinal cord tissue in the drug administration group is significantly more than that in the vehicle group, indicating that the plasminogen can enter the spinal cord tissue and be enriched in the spinal cord tissue. In addition, plasminogen is present in the cytoplasm and nucleus. Plasminogen and LAMP2 (red fluorescence) co-localize in the cytoplasm (as shown by Δ), and the expression level of LAMP2 in the spinal cord tissue in the drug administration group seems to be more than that in the vehicle group. This shows that plasminogen can enter the cell, co-localize with LAMP2, and promote the expression of LAMP2, promoting the action of lysosome.

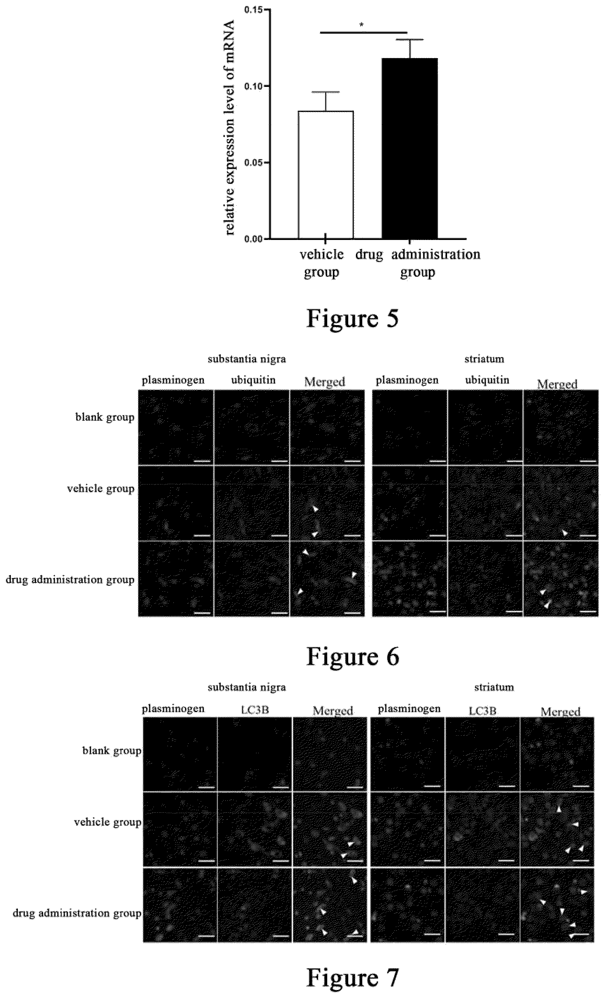

Figure 5 shows administration of plasminogen promotes the expression of LC3 in the spinal cord tissue of the amyotrophic lateral sclerosis model mice. The results show that the expression of LC3 in the drug administration group is significantly higher than that in the vehicle group, and the statistical difference is significant (* indicates P<0.05). This indicates that plasminogen can promote the transcription of the LC3 gene in the amyotrophic lateral sclerosis model mice, thereby participating in the regulation of the autophagy process.
Figure 6 shows the localization of plasminogen and the co-localization of plasminogen and ubiquitin in the substantia nigra and striatum tissues of the Parkinson's model mice. The results show that the positive staining of plasminogen (green fluorescence) in the substantia nigra and striatum tissues in the drug administration group is significantly more than that in the vehicle group, indicating that plasminogen can enter the substantia nigra and striatum tissues and be enriched in the substantia nigra and striatum tissues. **In** addition, plasminogen is present in the cytoplasm and nucleus. Plasminogen and ubiquitin (red fluorescence) co-localize in the cytoplasm (as shown by Δ). This shows that in the Parkinson's model mice, plasminogen can enter the substantia nigra and striatum tissues, enter the cells, and co-localize with ubiquitin. This suggests that plasminogen may interact with ubiquitin.
Figure 7 shows the localization of plasminogen and the co-localization of plasminogen and LC3B in the substantia nigra and striatum tissues of the Parkinson's model mice. The results show that the positive staining of plasminogen (green fluorescence) in the substantia nigra and striatum tissues in the drug administration group is significantly more than that in the vehicle group, indicating that plasminogen can enter the substantia nigra and striatum tissues and be enriched in the substantia nigra and striatum tissues. In addition, plasminogen is present in the cytoplasm and nucleus. Plasminogen and LC3B (red fluorescence) co-localize in the cytoplasm (as shown by Δ). This shows that plasminogen can enter the substantia nigra and striatum tissues, enter the cells, and co-localize with LC3B in the Parkinson's model mice. This suggests that plasminogen may interact with LC3.

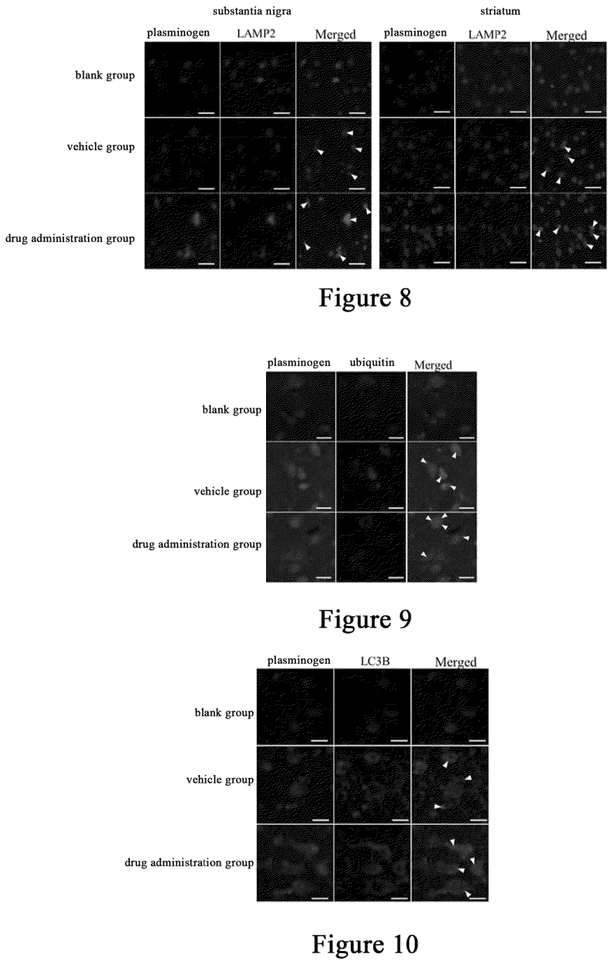

Figure 8 shows the localization of plasminogen and the co-localization of plasminogen and LAMP2 in the substantia nigra and striatum tissues of the Parkinson's model mice. The results show that the positive staining of plasminogen (green fluorescence) in the substantia nigra and striatum tissues in the drug administration group is significantly more than that in the vehicle group, indicating that the plasminogen can enter the substantia nigra and striatum tissues and be enriched in the substantia nigra and striatum tissues. In addition, plasminogen is present in the cytoplasm and nucleus. Plasminogen and LAMP2 (red fluorescence) co-localize in the cytoplasm (as shown by Δ). It shows that in the Parkinson's model mice, plasminogen can enter the substantia nigra and striatum tissues, enter the cells, and co-localize with LAMP2. It suggests that plasminogen may interact with LAMP2.
Figure 9 shows the localization of plasminogen and the co-localization of plasminogen and ubiquitin in the hippocampal issues of the Alzheimer's model mice. The results show that the positive staining of plasminogen (green fluorescence) in the hippocampal issues in the drug administration group is significantly more than that in the vehicle group, indicating that the plasminogen can enter the hippocampal issues and be enriched in the hippocampal issues. In addition, plasminogen is present in the cytoplasm and nucleus. Plasminogen and ubiquitin (red fluorescence) co-localize in the cytoplasm (as shown by Δ). This indicates that plasminogen can enter the hippocampal issues and enter the cells in the Alzheimer's model mice. This suggests that plasminogen may interact with ubiquitin.
Figure 10 shows the localization of plasminogen and the co-localization of plasminogen and LC3B in the hippocampal issues of the Alzheimer's model mice. The results show that the positive staining of plasminogen (green fluorescence) in the hippocampal issues in the drug administration group is significantly more than that in the vehicle group, indicating that the plasminogen can enter the hippocampal issues and be enriched in the hippocampal issues. In addition, plasminogen is present in the cytoplasm and nucleus. Plasminogen and LC3B (red fluorescence) co-localize in the cytoplasm (as shown by Δ). This indicates that plasminogen can enter the hippocampal issues and enter the cells in the Alzheimer's model mice. This suggests that plasminogen may interact with LC3B.

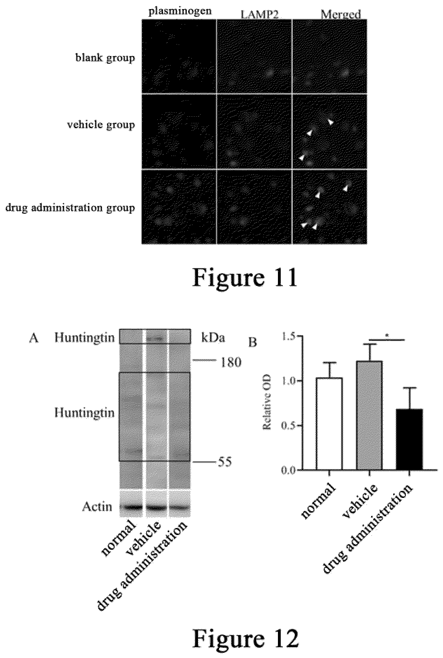

Figure 11 shows the localization of plasminogen and the co-localization of plasminogen and LAMP2 in the hippocampal issues of the Alzheimer's model mice. The results show that the positive staining of plasminogen (green fluorescence) in the hippocampal issues in the drug administration group is significantly more than that in the vehicle group, indicating that the plasminogen can enter the hippocampal issues and be enriched in the hippocampal issues. In addition, plasminogen is present in the cytoplasm and nucleus. Plasminogen and LAMP2 (red fluorescence) co-localize in the cytoplasm (as shown by Δ). This indicates that plasminogen can enter the hippocampal issues and enter the cells in the Alzheimer's model mice. This suggests that plasminogen may interact with LAMP2.
Figure 12A-B shows the results of western blot quantitative analysis of huntingtin in the heart tissue of the Huntington model mice 28 days after plasminogen administration. A is a western blot image, and B is the result of quantitative analysis of the optical density of the huntingtin band. The results show that the HTT level in the heart of the mice in the vehicle group is significantly higher than that of the mice in the normal control group, and the HTT level in the heart of the mice in the drug administration group is significantly lower than that of the mice in the vehicle group, and the statistical difference is significant (* represents P<0.05). This suggests that plasminogen can promote the degradation of huntingtin in the heart tissue of the Huntington model mice.

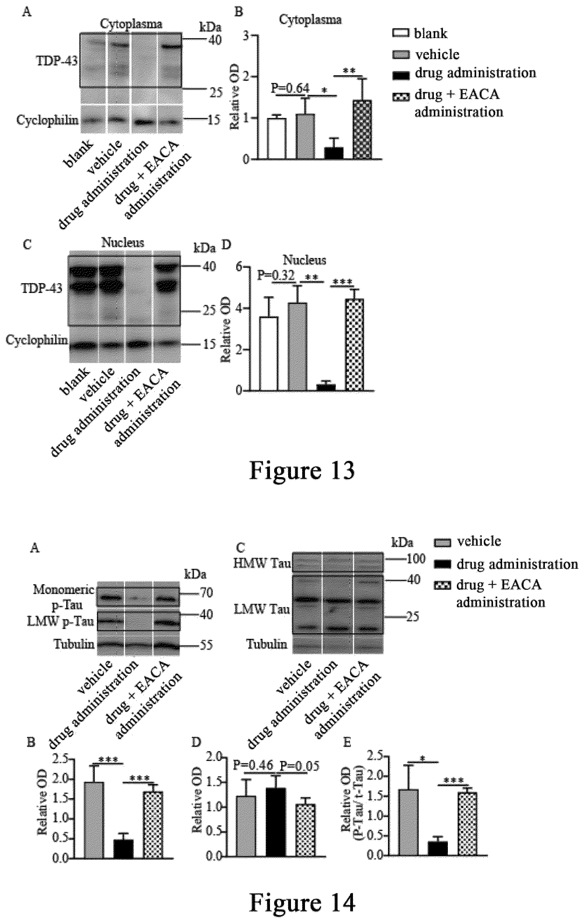

Figure 13 shows the results of WB detection of TDP-43 levels in the cytoplasm (A-B) and nucleus (C-D) of NSC34 cells treated with okadaic acid after administration of plasminogen. A and C are western blot images, and C and D are the results of quantitative analysis of TDP-43 bands. The results show that the levels of TDP-43 in the cytoplasm and nucleus in the drug administration group are significantly lower than those in the nucleus of the vehicle group, and the addition of EACA can completely inhibit the effect of plasminogen on TDP-43. * represents P<0.05, ** represents P<0.01, and *** represents P<0.001. This suggests that plasminogen can promote the degradation of TDP-43 in the cytoplasm and nucleus, and this effect of plasminogen is closely related to the lysine binding site in its structure.
Figure 14 shows the phosphorylated Tau protein (A-B), total Tau protein and the ratio of phosphorylated Tau protein to total Tau protein in NSC34 cells treated with okadaic acid after administration of plasminogen. A and C are western blot images, B, D and E are the results of phosphorylated Tau protein, total Tau protein and the ratio of phosphorylated Tau protein to total Tau protein, respectively. The results show that the level of the phosphorylated Tau protein in the drug administration group is significantly lower than that in the vehicle group, and the addition of EACA can completely inhibit the effect of plasminogen on the phosphorylated Tau protein; there is no significant difference between the total Tau protein level in the drug administration group and the vehicle group; the ratio of phosphorylated Tau protein to total Tau protein in the drug administration group is significantly lower than that in the vehicle group, and the addition of EACA can completely inhibit this effect of plasminogen. * represents P<0.05, *** represents P<0.001. It suggests that plasminogen can promote the degradation of phosphorylated Tau protein in cells, and this effect of plasminogen is closely related to the lysine binding site in its structure.

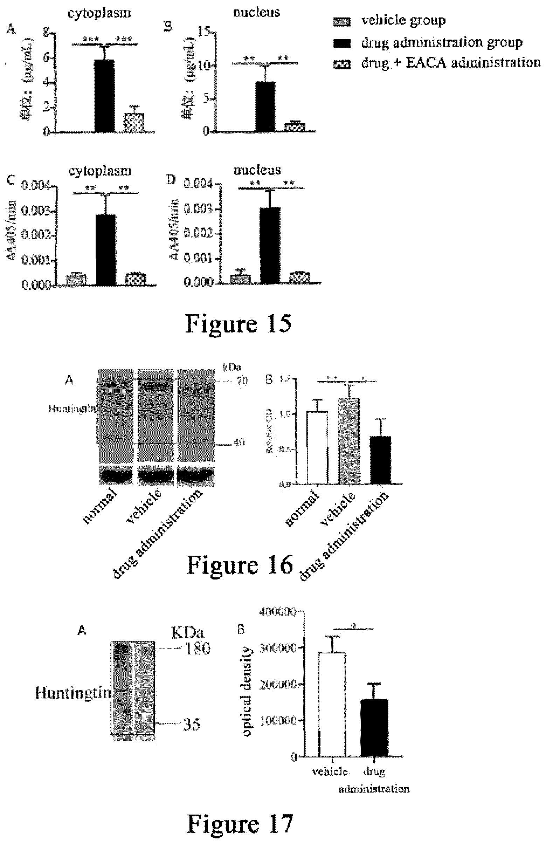

Figure 15A-D shows detection results of the plasminogen and plasmin activity levels in the cytoplasm and nucleus of NSC34 cells treated with okadaic acid after administration of plasminogen. A is detection results of the plasminogen level in the cytoplasm by the ELISA method, B is detection results of the plasminogen level in the nucleus by the ELISA method, C is detection results of the plasminogen level in the cytoplasm by the enzyme substrate kinetics method, and D is detection results of the plasminogen level in the nucleus by the enzyme substrate kinetics method. The results show that the levels of human plasminogen and fibrinolytic activity in the cytoplasm and nucleus in the drug administration group is significantly higher than that in the vehicle group, and the statistical difference is significant; and the addition of EACA can completely inhibit the effects of plasminogen (** represents P <0.01, *** represents P <0.001). It suggests that plasminogen can enter cells and even nucleus to increase plasmin activity, and the entry of plasminogen into cells and nucleus is closely related to its lysine binding activity.
Figure 16A-B shows the results of western blot quantitative analysis of huntingtin in the kidney tissue of Huntington model mice 28 days after plasminogen administration. A is a western blot image, and B is the result of quantitative analysis of the optical density of the huntingtin band. The results show that the HTT level in the kidney of the mice in the vehicle group is significantly higher than that of the mice in the normal control group, and the HTT level in the kidney of the mice of the drug administration group is significantly lower than that of the mice in the vehicle group (Figure 16A-B), and the statistical difference is significant (* represents P<0.05, *** represents P<0.001). This suggests that plasminogen can promote the degradation of huntingtin in the kidney tissue of the Huntington model mice.
Figure 17A-B shows the results of western blot quantitative analysis of huntingtin in the brain tissue of the Huntington model mice cultured in vitro 2 days after plasminogen administration. A is a western blot image, and B is the result of quantitative analysis of the optical density of the huntingtin band. The results show that the HTT level in the brain tissue of the mice in the drug administration group is significantly lower than that of the mice in the vehicle group, and the statistical difference is significant (* represents P<0.05). This suggests that plasminogen can promote the degradation of huntingtin in the brain tissue of the Huntington model mice.

### DETAIL DESCRIPTION OF THE INVENTION

Fibrinolytic system is a system consisting of a series of chemical substances involved in the process of fibrinolysis, which mainly includes plasminogen, plasmin, plasminogen activator, and fibrinolysis inhibitor. Plasminogen activators comprises tissue-type plasminogen activator (t-PA) and urokinase-type plasminogen activator (u-PA). t-PA is a serine protease that is synthesized by vascular endothelial cells. t-PA activates plasminogen, which is mainly carried out on fibrin; the urokinase-type plasminogen activator (u-PA) is produced by renal tubular epithelial cells and vascular endothelial cells, and may directly activate plasminogen without the need for fibrin as a cofactor. Plasminogen (PLG) is synthesized by the liver. When blood coagulates, a large amount of PLG is adsorbed on the fibrin network, and under the action of t-PA or u-PA it is activated into plasmin to promote fibrinolysis. Plasmin (PL) is a serine protease whose functions are as follows: degrading fibrin and fibrinogen; hydrolyzing various coagulation factors V, VIII, X, VII, XI, and **II,** etc.; converting plasminogen into plasmin; hydrolyzing complement, etc. Fibrinolysis inhibitors comprises plasminogen activator inhibitor (PAI) and α2 antiplasmin (α2-AP). PAI mainly has two forms, PAI-1 and PAI-2, which may specifically bind to t-PA in a ratio of 1:1, thereby inactivating it and activating PLG at the same time. α2-AP is synthesized by the liver, and binds to PL in a ratio of 1:1 to form a complex, thereby inhibiting the activity of PL; FXIII makes α2-AP covalently bound to fibrin, reducing the sensitivity of fibrin to PL. Substances that inhibit the activity of the fibrinolytic system *in vivo* comprise PAI-1, a complement C1 inhibitor; α2 antiplasmin; α2 macroglobulin.

The term "plasminogen pathway activator" herein encompasses a component of plasminogen activation pathway, a compound capable of directly activating plasminogen or indirectly activating plasminogen by activating a upstream component of plasminogen activation pathway, a compound mimicking the activity of plasminogen or plasmin, a compound capable of up-regulating the expression of plasminogen or an activator of plasminogen, an analog of plasminogen, an analog of plasmin, an analog of tPA or uPA, and an antagonist of a fibrinolysis inhibitor.

The term "component of plasminogen activation pathway" of the present application encompasses:
1. plasminogen, Lys-plasminogen, Glu-plasminogen, micro-plasminogen, delta-plasminogen;
   variants or analogs thereof;
2. plasmin and a variant or analog thereof; and
3. plasminogen activators, such as tPA and uPA, and tPA or uPA variants and analogs comprising one or more domains of tPA or uPA, such as one or more kringle domains and proteolytic domains.

The term "an antagonist of a fibrinolytic inhibitor" encompasses an antagonist of PAI-1, a complement C1 inhibitor, α2 antiplasmin or α2 macroglobulin, such as an antibody of PAI-1, a complement C1 inhibitor, α2 antiplasmin or α2 macroglobulin.

"Variants" of the above plasminogen, plasmin, tPA and uPA include all naturally occurring human genetic variants as well as other mammalian forms of these proteins, as well as a protein obtained by addition, deletion and/or substitution of such as 1-100, 1-90, 1-80, 1-70, 1-60, 1-50, 1-45, 1-40, 1-35, 1-30, 1-25, 1-20, 1-15, 1-10, 1-5, 1-4, 1-3, 1-2, or 1 amino acid (s), and still having the activity of plasminogen, plasmin, tPA or uPA. For example, "variants" of plasminogen, plasmin, tPA and uPA include mutational variants of these proteins obtained by substitution of such as 1-100, 1-90, 1-80, 1-70, 1-60, 1-50, 1- 45, 1-40, 1-35, 1-30, 1-25, 1-20, 1-15, 1-10, 1-5, 1-4, 1-3, 1-2, or 1 conservative amino acid (s).

A "plasminogen variant" of the application encompasses a protein comprising or having at least 75%, 80%, 85%, 90%, 95%, 96%, 97%, 98% or 99% sequence identity with an amino acid sequence of SEQ ID NO: 2, 6, 8, 10 or 12, and having plasminogen activity and/or lysine binding activity. For example, a "plasminogen variant" of the present application may be a protein obtained by addition, deletion and/or substitution of 1-100, 1-90, 1-80, 1-70, 1-60, 1-50, 1-45, 1-40, 1-35, 1-30, 1-25, 1-20, 1-15, 1-10, 1-5, 1-4, 1-3, 1-2, or 1 amino acid (s) on the basis of SEQ ID NO: 2, 6, 8, 10 or 12, and still having plasminogen activity and/or lysine binding activity. Particularly, the plasminogen variants of the present application include all naturally occurring human genetic variants as well as other mammalian forms of these proteins, as well as mutational variants of these proteins obtained by substitution of such as 1-100, 1-90, 1-80, 1-70, 1-60, 1-50, 1-45, 1-40, 1-35, 1-30, 1-25, 1-20, 1-15, 1-10, 1-5, 1-4, 1-3, 1-2, or 1 conservative amino acid (s).

The plasminogen of the present application may be a human plasminogen ortholog from a primate or rodent, or a variant thereof still retaining plasminogen activity and/or lysine binding activity, for example, a plasminogen represented by SEQ ID NO: 2, 6, 8, 10 or 12, such as a human natural plasminogen represented by SEQ ID NO: 2.

The "analogs" of the above plasminogen, plasmin, tPA, and uPA include compounds that provide substantially similar effects to plasminogen, plasmin, tPA, or uPA, respectively.

The "variants" and "analogs" of the above plasminogen, plasmin, tPA and uPA encompass "variants" and "analogs" of plasminogen, plasmin, tPA and uPA comprising one or more domains (e.g., one or more kringle domains and proteolytic domains). For example, "variants" and "analogs" of plasminogen encompass "variants" and "analogs" of plasminogen comprising one or more plasminogen domains (e.g., one or more kringle (k) domains and proteolytic domains, or called as serine protease domains, or plasminogen protease domains), such as mini-plasminogen. "Variants" and "analogs" of plasmin encompass "variants" and "analogs" of plasmin comprising one or more plasmin domains (e.g., one or more kringle domains and proteolytic domains), such as mini-plasmin, and delta-plasmin.

Whether a "variant" or "analog" of the above plasminogen, plasmin, tPA or uPA has the activity of plasminogen, plasmin, tPA or uPA respectively, or whether the "variant" or "analog" provides substantially similar effect to plasminogen, plasmin, tPA or uPA, may be detected by methods known in the art, for example, based on enzymography, ELISA (enzyme-linked immunosorbent assay), and FACS (fluorescence-activated cell sorting method), it is measured by the level of activated plasmin activity, for example, it is detected by referring to a method selected from the following documents: Ny, A., Leonardsson, G., Hagglund, A.C, Hagglof, P., Ploplis, V.A., Carmeliet, P. and Ny, T. (1999). Ovulation inplasminogen-deficient mice. Endocrinology 140, 5030-5035; Silverstein RL, Leung LL, Harpel PC, Nachman RL (November 1984). "Complex formation of platelet thrombospondin with plasminogen. Modulation of activation by tissue activator". J. Clin. Invest.74(5):1625-33; Gravanis I, Tsirka SE (February 2008). "Tissue-type plasminogen activator as a therapeutic target in stroke". Expert Opinion on Therapeutic Targets. 12(2):159-70; Geiger M, Huber K, Wojta J, Stingl L, Espana F, Griffin JH, Binder BR (Aug 1989). "Complex formation between urokinase and plasma protein C inhibitor in vitro and in vivo". Blood.74(2):722-8.

In some embodiments of the present application, the "component of plasminogen activation pathway" of the present application is a plasminogen selected from the group consisting of: Glu-plasminogen, Lys-plasminogen, mini-plasminogen, micro-plasminogen, delta-plasminogen, or variants thereof retaining plasminogen activity. In some embodiments, the plasminogen is a natural or synthetic human plasminogen, or a conservative mutant variant or fragment thereof still retaining plasminogen activity and/or lysine binding activity. In some embodiments, the plasminogen is a human plasminogen ortholog from a primate or rodent or a conservative mutant variant or fragment thereof still retaining plasminogen activity and/or lysine binding activity. In some embodiments, the amino acid sequence of the plasminogen comprises or has an amino acid sequence represented by SEQ ID NO: 2, 6, 8, 10 or 12. In some embodiments, the plasminogen is a human full length plasminogen. In some embodiments, the plasminogen is a human full length plasminogen represented by SEQ ID NO: 2.

"A compound capable of directly activating plasminogen, or indirectly activating plasminogen by activating a upstream component of plasminogen activation pathway", refers to any compound capable of directly activating plasminogen, or indirectly activating plasminogen by activating a upstream component of plasminogen activation pathway, such as tPA, uPA, streptokinase, saruplase, alteplase, reteplase, tenecteplase, anistreplase, monteplase, lanoteplase, pamiteplase, staphylokinase.

The "antagonist of a fibrinolysis inhibitor" of the present application is a compound that antagonizes, weakens, blocks, or prevents the action of the fibrinolysis inhibitor. Such fibrinolysis inhibitors are e.g., PAI-1, a complement C1 inhibitor, α2 antiplasmin, and α2 macroglobulin. Such an antagonist is: e.g., an antibody of PAI-1, a complement C1 inhibitor, α2 antiplasmin, or α2 macroglobulin; or an antisense RNA or small RNA blocking or down-regulating the expression of such as PAI-1, a complement C1 inhibitor, α2 antiplasmin or α2 macroglobulin; or a compound occupying the binding site of PAI-1, a complement C1 inhibitor, α2 antiplasmin, or α2 macroglobulin but without the function of PAI-1, a complement C1 inhibitor, α2 antiplasmin, or α2 macroglobulin; or a compound blocking the binding and/or active domains of PAI-1, a complement C1 inhibitor, α2 antiplasmin, or α2 macroglobulin.

Plasmin is a key component of the plasminogen activation system (PA system). It is a broad-spectrum protease capable of hydrolyzing several components of the extracellular matrix (ECM), including fibrin, gelatin, fibronectin, laminin, and proteoglycans. In addition, plasmin may activate some metalloproteinase precursors (pro-MMPs) to form active metalloproteinases (MMPs). Therefore, plasmin is considered to be an important upstream regulator of extracellular proteolysis. Plasmin is formed by proteolysis of plasminogen by two physiological PAs: tissue-type plasminogen activator (tPA) or urokinase-type plasminogen activator (uPA). Due to the relatively high levels of plasminogen in plasma and other body fluids, it has traditionally been believed that the regulation of the PA system is mainly achieved through the synthesis and activity levels of PAs. The synthesis of components of PA system is strictly regulated by different factors, such as hormone, growth factor and cytokine. In addition, there are specific physiological inhibitors of plasmin and PAs. The main inhibitor of plasmin is α2-antiplasmin. The activity of PAs is inhibited by plasminogen activator inhibitor-1 (PAI-1) of both uPA and tPA, and regulated by plasminogen activator inhibitor-2 (PAI-2) which mainly inhibits uPA. Certain cell surfaces have uPA-specific cell surface receptors (uPARs) with direct hydrolytic activity.

Plasminogen is a single-chain glycoprotein consisting of 791 amino acids with a molecular weight of approximately 92 kDa. Plasminogen is mainly synthesized in the liver, and is abundantly present in the extracellular fluid. The content of plasminogen in plasma is approximately 2 µM. Plasminogen is thus a huge potential source of proteolytic activity in tissues and body fluids. Plasminogen exists in two molecular forms: glutamate-plasminogen (Glu-plasminogen) and lysine-plasminogen (Lys-plasminogen). The naturally secreted and uncleaved form of plasminogen has an amino-terminal (N-terminal) glutamate, and is therefore referred to as glutamate-plasminogen. However, in the presence of plasmin, glutamate-plasminogen is hydrolyzed at Lys76-Lys77 into lysine-plasminogen. Compared with glutamate-plasminogen, lysine-plasminogen has a higher affinity for fibrin, and may be activated by PAs at a higher rate. The Arg560-Val561 peptide bond of these two forms of plasminogen may be cleaved by either uPA or tPA, resulting in the formation of double-chain protease plasmin linked by disulfide. The amino-terminal part of plasminogen comprises five homologous tri-cycles, i.e., so-called kringles, and the carboxy-terminal part comprises the protease domain. Some kringles comprise lysine-binding sites that mediate the specific interaction of plasminogen with fibrin and its inhibitor α2-AP. A recently found plasminogen is a 38 kDa fragment, including kringles1-4, and it is an effective inhibitor of angiogenesis. This fragment is named as angiostatin, and is produced by the hydrolysis of plasminogen by several proteases.

The main substrate of plasmin is fibrin, and the dissolution of fibrin is the key to preventing pathological thrombosis. Plasmin also has substrate specificity for several components of the ECM, including laminin, fibronectin, proteoglycans, and gelatin, indicating that plasmin also plays an important role in ECM reconstruction. Indirectly, plasmin may also degrade other components of the ECM, including MMP-1, MMP-2, MMP-3 and MMP-9, by converting certain protease precursors into active proteases. Therefore, it has been proposed that plasmin may be an important upstream regulator of extracellular proteolysis. In addition, plasmin has the ability to activate certain latent forms of growth factors. *In vitro,* plasmin also hydrolyzes components of the complement system, and releases chemotactic complement fragments.

"Plasmin" is a very important enzyme present in the blood that hydrolyzes fibrin clots into fibrin degradation products and D-dimers.

"Plasminogen" is the zymogen form of plasmin. According to the sequence in swiss prot, it consists of 810 amino acids calculating by the natural human plasminogen amino acid sequence (SEQ ID NO: 4) containing the signal peptide, and the molecular weight is about 90kD, and it is a glycoprotein mainly synthesized in the liver and capable of circulating in the blood, the cDNA sequence encoding this amino acid sequence is represented by SEQ ID NO: 3. Full-length plasminogen comprises seven domains: a C-terminal serine protease domain, an N-terminal Pan Apple (PAp) domain, and five Kringle domains (Kringle1-5). Referring to the sequence in swiss prot, its signal peptide comprises residues Met1-Gly19, PAp comprises residues Glu20-Val98, Kringle1 comprises residues Cys103-Cys181, Kringle2 comprises residues Glu184-Cys262, Kringle3 comprises residues Cys275-Cys352, Kringle4 comprises residues Cys377-Cys454, and Kringle5 comprises residues Cys481-Cys560. According to NCBI data, the serine protease domain comprises residues Val581-Arg804.

Glu-plasminogen is a natural full-length plasminogen, consisting of 791 amino acids (without a signal peptide of 19 amino acids); the cDNA sequence encoding this amino acid sequence is represented by SEQ ID NO: 1, and the amino acid sequence is represented by SEQ ID NO: 2. *In vivo,* there is also a Lys-plasminogen produced by the hydrolysis of amino acids 76 and 77 of Glu-plasminogen, as represented by SEQ ID NO: 6; and the cDNA sequence encoding this amino acid sequence is represented by SEQ ID NO: 5. Delta-plasminogen (δ-plasminogen) is a fragment of full-length plasminogen that lacks the Kringle2-Kringle5 structure, and only contains Kringle1 and a serine protease domain (also known as a proteolysis domain, or a plasminogen protease domain). The amino acid sequence of delta-plasminogen (SEQ ID NO: 8) is reported in a literature, and the cDNA sequence encoding this amino acid sequence is represented by SEQ ID NO: 7. Mini-plasminogen consists of Kringle5 and a serine protease domain, and it is reported that it comprises residues Val443-Asn791 (with the Glu residue of the Glu-plasminogen sequence without the signal peptide as the starting amino acid), its amino acid sequence is represented by SEQ ID NO: 10, and the cDNA sequence encoding this amino acid sequence is represented by SEQ ID NO: 9. While micro-plasminogen comprises only a serine protease domain, and it is reported that its amino acid sequence comprises residues Ala543-Asn791 (with the Glu residue of the Glu-plasminogen sequence without the signal peptide as the starting amino acid); additionally, it is disclosed in patent document CN102154253A that its sequence comprises residues Lys531-Asn791 (with the Glu residue of the Glu-plasminogen sequence without the signal peptide as the starting amino acid); the sequence of the present application refers to the patent document CN102154253A, the amino acid sequence is represented by SEQ ID NO: 12, and the cDNA sequence encoding this amino acid sequence is represented by SEQ ID NO: 11.

In the present application, "plasmin" and "fibrinolytic enzyme" may be used interchangeably with the same meaning; "plasminogen" and "fibrinolytic zymogen" may be used interchangeably with the same meaning.

In the present application, "lack" of plasminogen or plasminogen activity means that the content of plasminogen in a subject is lower than that of a normal person, and is sufficiently low to affect the normal physiological function of the subject; "deficiency" of plasminogen or plasminogen activity means that the content of plasminogen in a subject is significantly lower than that of a normal person, and even the activity or expression is extremely low, and the normal physiological function may only be maintained by external supply of plasminogen.

Those skilled in the art may understand that, all technical solutions of plasminogen of the present application are applicable to plasmin, thus the technical solutions described in the present application encompass plasminogen and plasmin. During circulation, plasminogen adopts a closed, inactive conformation, but when bound to a thrombus or cell surface, it is converted into active plasmin with an open conformation after being mediated by plasminogen activator (PA). Active plasmin may further hydrolyze the fibrin clot into degradation products of fibrin and D-dimers, thereby dissolving the thrombus. The PAp domain of plasminogen comprises an important determinant for maintaining plasminogen in an inactive closed conformation, while the KR domain may bind to a lysine residue present on a receptor and substrate. A variety of enzymes are known to act as plasminogen activators, including: tissue plasminogen activator (tPA), urokinase plasminogen activator (uPA), kallikrein, and coagulation factor XII (Hageman factor) etc.

"Active fragments of plasminogen" in this application include 1) in plasminogen protein, active fragments capable of binding to target sequences in substrates, also known as lysine-binding fragments, for example fragments comprising Kringle1, Kringle 2, Kringle 3, Kringle 4 and/or Kringle 5 (for the structure of the plasminogen, see Aisina RB, Mukhametova L I. Structure and function of plasminogen/plasmin system [J]. Russian Journal of Bioorganic Chemistry, 2014,40(6):590-605); 2) an active fragment that plays a proteolytic function in the plasminogen protein, e.g., a fragment comprising the plasminogen activity (proteolytic function) represented by SEQ ID NO: 14; 3) in the plasminogen protein, a fragment that has both the binding activity to the target sequence in the substrate (lysine binding activity) and the plasminogen activity (proteolytic function). In some embodiments of the present application, the plasminogen is a protein comprising the active fragment of plasminogen represented by SEQ ID NO: 14. In some embodiments of the present application, the plasminogen is a protein comprising a lysine-binding fragment of Kringle 1, Kringle 2, Kringle 3, Kringle 4 and/or Kringle 5. In some embodiments, the plasminogen active fragment of the present application comprises SEQ ID NO: 14, or a protein with an amino acid sequence having at least 80%, 90%, 95%, 96%, 97%, 98%, 99% homology with SEQ ID NO: 14. Therefore, the plasminogen of the present application comprises a protein comprising the active fragment of the plasminogen and still maintaining the activity of the plasminogen. In some embodiments, the plasminogen of the present application comprises Kringle 1, Kringle 2, Kringle 3, Kringle 4 and/or Kringle 5, or a protein has at least 80%, 90%, 95%, 96%, 97%, 98%, 99% homologous with Kringle 1, Kringle 2, Kringle 3, Kringle 4 and/or Kringle 5 and still have lysine-binding activity.

At present, the methods for measuring plasminogen and its activity in blood comprise: detection of tissue plasminogen activator activity (t-PAA), detection of plasma tissue plasminogen activator antigen (t-PAAg), detection of plasma tissue plasminogen activity (plgA), detection of plasma tissue plasminogen antigen (plgAg), detection of the activity of plasma tissue plasminogen activator inhibitor, detection of the antigen of plasma tissue plasminogen activator inhibitor, and detection of plasma plasmin-antiplasmin complex (PAP); wherein the most commonly used detection method is the chromogenic substrate method: adding streptokinase (SK) and a chromogenic substrate to the plasma to be detected, the PLG in the plasma to be detected is converted into PLM under the action of SK, and PLM acts on the chromogenic substrate; subsequently, the detection by spectrophotometer indicates that the increase in absorbance is proportional to plasminogen activity. In addition, the plasminogen activity in blood may also be detected by immunochemical method, gel electrophoresis, immunoturbidimetry, and radioimmunoassay, etc.

"Ortholog or orthologs" refer to homologs between different species, including both protein homologs and DNA homologs, also known as orthologs and vertical homologs; particularly it refers to proteins or genes evolved from the same ancestral gene in different species. The plasminogen of the present application includes human natural plasminogen, and also includes plasminogen ortholog or orthologs derived from different species and having plasminogen activity.

A "conservative substitution variant" refers to a variant in which a given amino acid residue is altered without changing the overall conformation and function of the protein or enzyme, including but not limited to those variants in which the amino acid(s) in the amino acid sequence of the parent protein are replaced by amino acid(s) with similar properties (e.g., acidic, basic, hydrophobic, etc.). Amino acids with similar properties are well known in the art. For example, arginine, histidine and lysine are hydrophilic basic amino acids and are interchangeable. Similarly, isoleucine is a hydrophobic amino acid, and may be replaced by leucine, methionine or valine. Therefore, the similarity of two proteins or amino acid sequences with similar functions may differ; for example, 70% to 99% similarity (identity) based on the MEGALIGN algorithm. "Conservative substitution variants" also include polypeptides or enzymes having more than 60%, preferably more than 75%, more preferably more than 85%, or even most preferably more than 90% amino acid identity determined by BLAST or FASTA algorithm, and having the same or substantially similar properties or functions as the natural or parent protein or enzyme.

"Isolated" plasminogen refers to a plasminogen protein isolated and/or recovered from its natural environment. **In** some embodiments, the plasminogen will be purified: (1) to more than 90%, more than 95%, or more than 98% purity (by weight), as determined by Lowry's method, e.g., more than 99% (by weight), (2) to a degree sufficient to obtain at least 15 residues of the N-terminal or internal amino acid sequence by using a spinning cup sequence analyzer, or (3) to homogeneity as determined by sodium dodecyl sulfate-polyacrylamide gel electrophoresis (SDS-PAGE) under reducing or nonreducing conditions by using Coomassie blue or silver staining. Isolated plasminogen also includes plasminogen prepared from recombinant cells by bioengineering techniques and isolated by at least one purification step.

The terms "polypeptide", "peptide" and "protein" are used interchangeably herein to refer to a polymeric form of amino acids of any length, which may include genetically encoded and non-genetically encoded amino acids, chemically or biochemically modified or derivatized amino acids, and polypeptides with modified peptide backbones. The terms include fusion proteins including, but not limited to, fusion proteins with heterologous amino acid sequences, fusions with heterologous and homologous leader sequences (with or without N-terminal methionine residues); and the like.

"Percent (%) of amino acid sequence identity" with respect to a reference polypeptide sequence is defined as, after introducing gaps as necessary to achieve maximum percent sequence identity, and no conservative substitutions are considered as a part of the sequence identity, the percentage of amino acid residues in a candidate sequence that are identical to the amino acid residues in a reference polypeptide sequence. Alignment for purposes of determining percent amino acid sequence identity may be accomplished in a variety of ways within the technical scope in the art, e.g., by using publicly available computer software, such as BLAST, BLAST-2, ALIGN or Megalign (DNASTAR) software. Those skilled in the art may determine the appropriate parameters for aligning sequences, including any algorithms needed to achieve maximal alignment over the full length of the sequences to be compared. However, for the purpose of the present application, the values of percent amino acid sequence identity are generated by using the sequence comparison computer program ALIGN-2.

Where ALIGN-2 is used to compare amino acid sequences, the percentage (%) of amino acid sequence identity of a given amino acid sequence A relative to a given amino acid sequence B (or may be expressed as a given amino acid sequence A having or comprising a certain percentage (%) of amino acid sequence identity relative to, with or with respective to a given amino acid sequence B) is calculated as follows:
Fraction X/Y times 100;
wherein X is the number of amino acid residues scored as identical matches during the alignment of sequences A and B by the sequence alignment program ALIGN-2, and wherein Y is the total number of amino acid residues in sequence B. It should be appreciated that, where the length of amino acid sequence A is not equal to that of amino acid sequence B, the percentage (%) of amino acid sequence identity of A with respect to B will not equal to the percentage (%) of amino acid sequence identity of B with respect to A. Unless expressly stated otherwise, all the values of percentage (%) of amino acid sequence identity used herein are obtained by using the ALIGN-2 computer program as described in the preceding paragraph.

The terms "individual", "subject" and "patient" are used interchangeably herein to refer to mammals including, but not limited to, murine (rat, mouse), non-human primate, human, canine, feline, hoofed animals (e.g., horses, cattle, sheep, pigs, goats), etc.

A "therapeutically effective amount" or "effective amount" refers to an amount of plasminogen sufficient to prevent and/or treat a disease when administrated to a mammal or other subject for treating the disease. The "therapeutically effective amount" will vary depending on the plasminogen used, the severity of the disease and/or symptoms thereof, as well as the age, body weight of the subject to be treated, and the like.

The term "treatment" of a disease state includes inhibiting or preventing the development of the disease state or its clinical symptoms, or alleviating the disease state or symptoms, resulting in temporary or permanent regression of the disease state or its clinical symptoms.

### Preparation of the Plasminogen of the Present Application

Plasminogen may be isolated and purified from nature for further therapeutic use, or it may be synthesized by standard chemical peptide synthesis techniques. When the polypeptide is synthesized chemically, the synthesis may be carried out via liquid phase or solid phase. Solid-phase polypeptide synthesis (SPPS) (in which the C-terminal amino acid of the sequence is attached to an insoluble support, followed by the sequential addition of the retaining amino acids in the sequence) is a suitable method for chemical synthesis of plasminogen. Various forms of SPPS, such as Fmoc and Boc, may be used to synthesize plasminogen. Techniques for solid-phase synthesis are described in Barany and Solid-Phase Peptide Synthesis; pp.3-284 in The Peptides: Analysis, Synthesis, Biology. Vol. 2: Special Methods in Peptide Synthesis, Part A., Merrifield, et al. J. Am. Chem. Soc., 85:2149-2156 (1963); Stewart et al., Solid Phase Peptide Synthesis, 2nd ed. Pierce Chem. Co., Rockford, Ill. (1984); and Ganesan A. 2006 Mini Rev. Med Chem. 6:3-10, and Camarero JA et al. 2005, Protein Pept Lett. 12:723-8. Briefly, small insoluble porous beads are treated with functional units on which peptide chains are constructed; after repeated cycles of coupling/deprotection, the attached solid-phase free N-terminal amine is coupled to a single N-protected amino acid unit. This unit is then deprotected to expose new N-terminal amines that may be attached to other amino acids. The peptide remains immobilized on the solid phase, subsequently it is cleaved off.

Plasminogen of the present application may be produced by standard recombinant methods. For example, a nucleic acid encoding plasminogen is inserted into an expression vector to operably link to regulatory sequences in the expression vector. The regulatory sequences for expression include, but are not limited to, promoters (e.g., naturally associated or heterologous promoters), signal sequences, enhancer elements, and transcription termination sequences. Expression regulation may be a eukaryotic promoter system in a vector capable of transforming or transfecting a eukaryotic host cell (e.g., COS or CHO cell). Once the vector is incorporated into a suitable host, the host is maintained under conditions suitable for high-level expression of the nucleotide sequence and collection and purification of plasminogen.

A suitable expression vector typically replicates in a host organism as an episome or as an integrated part of the host chromosomal DNA. Typically, an expression vector contains a selectable marker (e.g., ampicillin resistance, hygromycin resistance, tetracycline resistance, kanamycin resistance, or neomycin resistance marker) to facilitate the detection of those cells transformed with desired exogenous DNA sequence.

*Escherichia coli* is an example of a prokaryotic host cell that may be used to clone a plasminogen-encoding polynucleotide. Other microbial hosts suitable for use include bacilli such as *Bacillus subtilis,* and other *enterobacteriaceae* such as *Salmonella, Serratia,* and various *Pseudomonas* species. In these prokaryotic hosts, expression vectors may also be generated, which will typically contain an expression control sequence (e.g., origin of replication) that are compatible with the host cell. In addition, there are many well-known promoters, such as the lactose promoter system, the tryptophan (trp) promoter system, the beta-lactamase promoter system, or the promoter system from bacteriophage lambda. A promoter will typically control the expression, optionally in case of an operator gene sequence, and have ribosome binding site sequence, etc., to initiate and complete transcription and translation.

Other microorganisms, such as yeast, may also be used for expression. Yeast (e.g., *Saccharomyces cerevisiae (S. cerevisiae))* and *Pichia* are examples of suitable yeast host cells, and as required a suitable vector has an expression control sequence (e.g., promoter), origin of replication, termination sequence, etc. A typical promoter comprises 3-phosphoglycerate kinase and other saccharolytic enzymes. Particularly, inducible yeast promoters include promoters from ethanol dehydrogenase, isocytochrome C, and enzymes responsible for maltose and galactose utilization.

In addition to microorganisms, mammalian cells (e.g., mammalian cells cultured in *in vitro* cell culture) may also be used to express and produce the plasminogen of the application (e.g., polynucleotides encoding the plasminogen). See Winnacker, From Genes to Clones, VCH Publishers, N.Y., N.Y. (1987). Suitable mammalian host cells include CHO cell lines, various Cos cell lines, HeLa cells, myeloma cell lines, and transformed B cells or hybridomas. Expression vectors for use in these cells may comprise expression control sequences such as origin of replication, promoter and enhancer (Queen et al., Immunol. Rev. 89:49 (1986)), and necessary sites for processing information such as ribosome binding sites, RNA splicing sites, polyadenylation sites, and transcription terminator sequences. Examples of suitable expression control sequences are promoters derived from immunoglobulin gene, SV40, adenovirus, bovine papilloma virus, cytomegalovirus, and the like. See Co et al, J. Immunol. 148:1149 (1992).

Once synthesized (chemically or recombinantly), the plasminogen of the present application may be purified according to standard procedures in the art, including ammonium sulfate precipitation, affinity column, column chromatography, high performance liquid chromatography (HPLC), gel electrophoresis, and the like. The plasminogen is substantially pure, e.g., at least about 80-85% pure, at least about 85-90% pure, at least about 90-95% pure, or 98-99% pure or purer, e.g., free of contaminants such as cellular debris, macromolecules other than the plasminogen, and the like.

### Pharmaceutical Formulation

A therapeutic formulation may be prepared by mixing the plasminogen of desired purity with an optional pharmaceutical carrier, excipient, or stabilizer (Remington's Pharmaceutical Sciences, 16th edition, Osol, A. ed. (1980)), to form a lyophilized formulation or aqueous solution. An acceptable carrier, excipient, or stabilizer is non-toxic to a recipient at the employed dosage and concentration, including buffers such as phosphate, citrate and other organic acids; antioxidants including ascorbic acid and methionine; preservatives (such as octadecyldimethylbenzylammonium chloride; hexanediamine chloride; benzalkonium chloride, benzethonium chloride; phenol, butanol or benzyl alcohol; alkyl parahydroxybenzoate such as methyl or propyl paraben; catechol; resorcinol; cyclohexanol; 3-pentanol; m-cresol); low molecular weight polypeptides (less than about 10 residues); proteins such as serum albumin, gelatin or immunoglobulins; hydrophilic polymers such as polyvinylpyrrolidone; amino acids such as glycine, glutamine, asparagine, histidine, arginine or lysine; monosaccharides, disaccharides and other carbohydrates including glucose, mannose, or dextrin; chelating agents such as EDTA; carbohydrates such as sucrose, mannitol, fucose, or sorbitol; salt-forming counterions such as sodium; metal complexes (such as zinc-protein complexes); and/or nonionic surfactants such as TWENTM, PLURONICSTM or polyethylene glycol (PEG).

The formulations of the present application may also contain more than one active compound as required for the particular condition to be treated, preferably those compounds are complementary in activity and do not have side effects with each other, for example, antihypertensive medicaments, antiarrhythmic medicaments, medicaments for treating diabetes, etc.

The plasminogen of the present application may be encapsulated in microcapsules prepared by techniques such as coacervation or interfacial polymerization, for example, the plasminogen may be placed in colloidal drug delivery systems (e.g., liposomes, albumin microspheres, microemulsions, nanoparticles and nanocapsules) or in hydroxymethyl cellulose or gel-microcapsules and poly-(methyl methacrylate) microcapsules in macroemulsions. These techniques are disclosed in Remington's Pharmaceutical Sciences 16th edition, Osol, A. Ed. (1980).

The plasminogen of the present application for *in vivo* administration must be sterile. This may be easily achieved by filtration through sterilizing filters before or after lyophilization and reformulation.

The plasminogen of the present application may be prepared as a sustained-release formulation. Suitable examples of sustained-release formulations include semipermeable matrices of solid hydrophobic polymers which have a certain shape and contain glycoprotein, for example, membranes or microcapsules. Examples of sustained-release matrices include polyesters, hydrogels such as poly(2-hydroxyethyl-methacrylate) (Langer et al., J. Biomed. Mater. Res., 15:167-277 (1981); Langer, Chem. Tech., 12:98-105 (1982)), or poly(vinyl alcohol), polylactide (US Pat. No.3,773,919, EP58,481), copolymers of L-glutamic acid and γ-ethyl-L-glutamic acid (Sidman, et al., Biopolymers 22:547 (1983)), non-degradable ethylene-vinyl acetate (Langer, et al., *supra*), or degradable lactic acid-glycolic acid copolymers such as Lupron Depot^{™} (injectable microspheres consisting of lactic acid-glycolic acid copolymer and leuprolide acetate), and poly-D-(-)-3-hydroxybutyric acid. Polymers such as ethylene-vinyl acetate and lactic acid-glycolic acid may release molecules continuously for more than 100 days, while some hydrogels release proteins for shorter period of time. Rational strategies to stabilize proteins may be devised based on the relevant mechanisms. For example, if the mechanism of condensation is found to form intermolecular S-S bond through thiodisulfide interchange, then stabilization may be achieved by modifying sulfhydryl residues, lyophilizing from acidic solutions, controlling humidity, using suitable additives, and developing specific polymer matrix compositions.

### Administration and Dosage

Administration of the pharmaceutical composition of the present application may be accomplished by different means, e.g., intravenously, intraperitoneally, subcutaneously, intracranially, intrathecally, intraarterally (e.g., via the carotid artery), and intramuscularly.

Preparations for parenteral administration include sterile aqueous or non-aqueous solutions, suspensions and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, or fixed oils. Intravenous vehicles include fluid and nutritional supplements, electrolyte supplements, and the like. Preservatives and other additives may also be present, such as, for example, antimicrobials, antioxidants, chelating agents, and inert gases, etc.

Dosing regimens will be determined by medical personnel based on various clinical factors. As is well known in the medical field, the dosage for any patient depends on a variety of factors, including the patient's size, body surface area, age, the particular compound to be administrated, sex, number and route of administration, general health, and other medicaments administrated concomitantly. The dosage range of the pharmaceutical composition comprising the plasminogen of the present application may be about 0.0001-2000 mg/kg, or about 0.001-500 mg/kg (e.g., 0.02 mg/kg, 0.25 mg/kg, 0.5 mg/kg, 0.75 mg/kg, 10 mg/kg, 50 mg/kg, etc.) body weight of the subject per day. For example, the dose may be 1 mg/kg body weight, or 50 mg/kg body weight, or in the range of 1-50 mg/kg, or at least 1 mg/kg. Dosages above or below this exemplary range are also contemplated, especially in view of the factors set forth above. Intermediate doses within the above ranges are also included within the scope of the present application. Subjects may be administrated such doses daily, every other day, weekly, or according to any other schedule determined by empirical analysis. An exemplary dosage schedule includes 1-10 mg/kg for consecutive days. Real-time evaluation of therapeutic efficacy and safety is required during the administration of the medicament of the present application.

### A Product or Kit

One embodiment of the present application relates to a product or kit comprising the plasminogen or plasmin of the present application for treating cardiovascular disease and related disorders caused by diabetes. The product preferably comprises a container, and a label or package insert. Suitable containers are bottles, vials, syringes, etc. The container may be made of various materials such as glass or plastic. The container contains a composition which is effective for treatment of the disease or disorder of the present application and has a sterile access port (e.g., the container may be an intravenous solution pack or vial containing a stopper penetrable by a hypodermic injection needle). At least one active agent in the composition is plasminogen/plasmin. The label on or attached to the container indicates that the composition is used for treatment of cardiovascular disease and related disorders caused by diabetes of the present application. The product may further comprise a second container containing a pharmaceutically acceptable buffer, such as phosphate buffered saline, Ringer's solution, and dextrose solution. It may further contain other substances required from a commercial and user standpoint, including other buffers, diluents, filters, needles and syringes. In addition, the article of manufacture comprises a package insert with instructions for use, including, for example, instructing the user of the composition to administrate the plasminogen composition along with other medicaments for treatment of concomitant diseases to the patient.

In some embodiments, the above disease caused by pathological protein aggregation includes: Alzheimer's disease, Parkinson's disease, amyotrophic lateral sclerosis, prion-like disease (Creutzfeldt-Jakob disease), Gerstmann syndrome, dispersive or familial fatal insomnia and kuru disease, Huntington's disease, spinocerebellar ataxia type 3, and dentatorubral-pallidoluysian atrophy.

In some embodiments, the above disease caused by ubiquitin/lysosome dysfunction includes: Fanconi anemia, Xeroderma pigmentosum, Cockayne syndrome, cancer, Cowden syndrome, Parkinson's disease, genomic instability, metabolic syndrome, myatrophy, Von Hippel Lindau syndrome, multiple myeloma, hereditary pseudo hyperaldosteronism (RIDDLE syndrome), Huntington's disease, X-linked lymphoproliferative disease, Crohn's disease, Alzheimer's disease, breast cancer and ovarian cancer, muscular amylopectinosis, amyotrophic lateral sclerosis, spinal muscular atrophy, systemic lupus erythematosus, childhood ataxia, X-linked parkinsonism syndrome with spasticity, multisystem disorder, diabetes, multiple sclerosis, cystinosis, Vici syndrome, Gaucher's disease, frontotemporal dementia (heterozygous) or neuronal ceroid lipofuscinosis (homozygous), Danon's cardiomyopathy, cortical atrophy, epilepsy, autosomal recessive spinal and cerebellar ataxia, hereditary spastic paraplegia, beta-propeller protein-associated neurodegeneration (BPAN), autism spectrum disorder, frontotemporal dementia (FTD), inflammatory bowel disease, nonalcoholic fatty liver disease, pulmonary tuberculosis, Rett syndrome, osteopetrosis, Charcot-Marie-Tooth type 2B disease, juvenile arthritis, Snyder-Robinson syndrome (SRS), Wiskott-Aldrich syndrome, primary microcephaly, hereditary sensory and autonomic neuropathy type II, primary open angle glaucoma (POAG), Paget's disease of the bone (PGD), colon cancer, lung cancer, brain cancer, autosomal recessive and sporadic early-onset Parkinson's disease, Zellweger syndrome spectrum disorders, distal myopathy, ulcerative colitis, familial Mediterranean fever, ataxia with spasticity, Fabry disease, Gaucher disease, lysosomal acid lipase deficiency, mucopolysaccharidosis, and Angelman syndrome.

### Example

The human plasminogen used in all the following examples was from plasma of the donor, and was obtained by purified from plasma of the human donor based on the method described in the literature [1-3] (Kenneth C Robbins, Louis Summaria, David Elwyn et al. Further Studies on the Purification and Characterization of Human Plasminogen and Plasmin. Journal of Biological Chemistry, 1965, 240 (1): 541-550; Summaria L, Spitz F, Arzadon L et al. Isolation and characterization of the affinity chromatography forms of human Glu- and Lys-plasminogens and plasmins. J Biol Chem. 1976 Jun 25; 251 (12): 3693-9; HAGAN JJ, ABLONDI FB, DE RENZO EC. Purification and biochemical properties of human plasminogen. J Biol Chem. 1960 Apr; 235: 1005-10) and process optimization, wherein the purity of human Lys-plasminogen and Glu-plasminogen > 98%.

### Example 1 Plasminogen is enriched and co-localized with ubiquitin in the spinal cord tissue of the ALS model mice

Transgenic mutant SOD1 has the histopathological characteristics observed in the clinic of sporadic and familial amyotrophic lateral sclerosis (ALS). The ALS model mice in this case were B6.Cg-Tg(SOD1-G93A)1Gur/J transgenic mice (referred to as SOD1-G93A), and purchased from Jackson Laboratory, with a pedigree number of 004435, and animal-related experiments were conducted in an SPF grade environment. SOD1-G93A model mice have been widely used in the mechanism research of ALS and preclinical experimental research of new drug development.

Five wild-type male mice and nine male SOD1-G93A mice of similar age were taken. Wild-type mice were used as the blank control group. SOD1-G93A mice were observed and recorded from the 14th week when their hind legs trembled after the onset of the disease. The onset time of each mouse was recorded. Drug administration began 14 days after the onset of the disease. All mice were randomly divided into a vehicle group and a drug administration group according to the onset of the disease. Among them, there were 5 mice in the vehicle group, and 0.1 ml/mouse vehicle (sodium citrate buffer) was injected into the tail vein every day; there were 4 mice in the drug administration group, and 1 mg/0.1 ml/mouse plasminogen was injected into the tail vein every day. The drug administration was continuous under SPF environment, and the samples were collected at the end of death. The longest drug administration was 61 days. The spinal cord tissue was fixed in formalin fixing solution. The fixed tissue was subjected to gradient dehydration with alcohol and cleared with xylene before paraffin embedding. The thickness of the tissue section was 3 µm, and the sections were washed once after dewaxing and rehydration. The sections were immersed in the antigen retrieval working solution (0.01M sodium citrate buffer) and microwave repaired, preheating for 5 minutes, high heat for 2 minutes, and low heat for 15 minutes. The tissue was circled with a PAP pen, incubated with 3% hydrogen peroxide for 15 minutes, and washed twice with 0.01M PBS for 5 minutes each time. 5% of normal goat serum (Vector laboratories, Inc., USA) was used for blocking for 30 minutes. After the time was up, the goat serum was discarded, anti-plasminogen antibody (self-produced) was added dropwise, incubated overnight at 4°C, and washed twice with 0.01M PBS for 5 minutes each time. The goat anti-rabbit IgG (HRP) antibody (Abcam) as a secondary antibody was incubated at room temperature for 1 hour, and washed twice with 0.01M PBS for 5 minutes each time. According to the instructions of the XTSA520IHC kit (Alpha X Biotech, AXT6202500), the green fluorescence color development of the corresponding anti-plasminogen secondary antibody was performed. It was washed 3 times with PBS for 5 minutes each time. The above antigen retrieval and blocking operations were repeated, and then staining with anti-ubiquitin antibody (Abcam, ab7780) was performed, incubating at 37°C for 1 hour. It was washed 3 times with PBS for 5 minutes each time. The goat anti-rabbit IgG (HRP) antibody (Abcam) as a secondary antibody was incubated at room temperature for 1 hour, and washed twice with 0.01M PBS for 5 minutes each time. According to the instructions of the XTSA620IHC kit (Alpha X Biotech, AXT6502500), the red fluorescence color development of the corresponding anti-ubiquitin secondary antibody was performed. It was washed 3 times with PBS for 5 minutes each time. Nuclear staining with DAPI (BOSTER, 11K16B77) was performed. It was washed 3 times with PBS for 5 minutes each time. The slice was dehydrated with gradient ethanol, cleared with xylene, sealed by neutral gum, observed and photographed under a 400x optical microscope.

Ubiquitin (Ub) is a highly conserved small molecular weight protein with a molecular weight of about 8,500. It is composed of 76 amino acids and is widely present in eukaryotic cells. The main function of ubiquitin is to participate in the degradation of most proteins in eukaryotic cells, and the proteasome is the site of ubiquitin-mediated protein degradation. The ubiquitin-proteasome pathway is one of the important ways to "digest" intracellular proteins.

The results show that the positive staining of plasminogen (green fluorescence) in the spinal cord tissue in the drug administration group is significantly more than that in the vehicle group, indicating that the plasminogen can enter the spinal cord tissue and be enriched in the spinal cord tissue. In addition, plasminogen is present in the cytoplasm and nucleus. Plasminogen and ubiquitin (red fluorescence) co-localize in the cytoplasm (as shown by Δ) (Figure 2), and the ubiquitin expression level in the spinal cord tissue in the drug administration group seems to be less than that in the vehicle group. This shows that plasminogen can enter the spinal cord tissue, enter the cell, co-localize with ubiquitin, and reduce the expression of ubiquitin in the amyotrophic lateral sclerosis model mice. This suggests that plasminogen may interact with ubiquitin.

### Example 2 Plasminogen is enriched and co-localized with LC3B in the spinal cord tissue of the ALS model mice

Five wild-type male mice and nine male SOD1-G93A mice of similar age were taken. Wild-type mice were used as the blank control group. SOD1-G93A mice were observed and recorded from the 14th week when their hind legs trembled after the onset of the disease. The onset time of each mouse was recorded. Drug administration began 14 days after the onset of the disease. All mice were randomly divided into a vehicle group and a drug administration group according to the onset of the disease. Among them, there were 5 mice in the vehicle group, and 0.1 ml/mouse vehicle (sodium citrate buffer) was injected into the tail vein every day; there were 4 mice in the drug administration group, and 1 mg/0.1 ml/mouse plasminogen was injected into the tail vein every day. The drug administration was continuous under SPF environment, and the samples were collected at the end of death. The longest drug administration was 61 days. The spinal cord tissue was fixed in formalin fixing solution. The fixed tissue was subjected to gradient dehydration with alcohol and cleared with xylene before paraffin embedding. The thickness of the tissue section was 3 µm, and the sections were washed once after dewaxing and rehydration. The sections were immersed in the antigen retrieval working solution (0.01M sodium citrate buffer) and microwave repaired, preheating for 5 minutes, high heat for 2 minutes, and low heat for 15 minutes. The tissue was circled with a PAP pen, incubated with 3% hydrogen peroxide for 15 minutes, and washed twice with 0.01M PBS for 5 minutes each time. 5% of normal goat serum (Vector laboratories, Inc., USA) was used for blocking for 30 minutes. After the time was up, the goat serum was discarded, anti-plasminogen antibody (self-produced) was added dropwise, incubated overnight at 4°C, and washed twice with 0.01M PBS for 5 minutes each time. The goat anti-rabbit IgG (HRP) antibody (Abcam) as a secondary antibody was incubated at room temperature for 1 hour, and washed twice with 0.01M PBS for 5 minutes each time. According to the instructions of the XTSA520IHC kit (Alpha X Biotech, AXT6202500), the green fluorescence color development of the corresponding anti-plasminogen secondary antibody was performed. It was washed 3 times with PBS for 5 minutes each time. The above antigen retrieval and blocking operations were repeated, and then staining with anti-LC3B antibody (Proteintech, 18725-1-AP) was performed, incubating at 37°C for 1 hour. It was washed 3 times with PBS for 5 minutes each time. The goat anti-rabbit IgG (HRP) antibody (Abcam) as a secondary antibody was incubated at room temperature for 1 hour, and washed twice with 0.01M PBS for 5 minutes each time. According to the instructions of the XTSA620IHC kit (Alpha X Biotech, AXT6502500), the red fluorescence color development of the corresponding anti-LC3B secondary antibody was performed. It was washed 3 times with PBS for 5 minutes each time. Nuclear staining with DAPI (BOSTER, 11K16B77) was performed. It was washed 3 times with PBS for 5 minutes each time. The slice was dehydrated with gradient ethanol, cleared with xylene, sealed by neutral gum, observed and photographed under a 400x optical microscope.

Autophagy is a process of degrading dysfunctional cellular components inside cells through lysosomes. Autophagy can degrade and digest damaged and denatured organelles, proteins, nucleic acids and other biomacromolecules. It provides raw materials for cell regeneration and repair, and realizes the recycling of intracellular substances. LC3 refers to protein light chain 3, which is a marker of the autophagic process. Its function is mainly involved in the formation of autophagosomes. The LC3 precursor molecule is cleaved to remove the 5-peptide at the C-terminal to form LC3-I in a cytoplasmic form. It is then activated by APG7L/ATG7, transferred to ATG3 and coupled with phosphatidyl ethanolamine (PE) to form LC3-II (LC3B) in a membrane-bound form, which can attach to the membrane of the autophagosome and is a structural protein of the autophagosome.

The results show that the positive staining of plasminogen (green fluorescence) in the spinal cord tissue in the drug administration group is significantly more than that in the vehicle group, indicating that the plasminogen can enter the spinal cord tissue and be enriched in the spinal cord tissue. In addition, plasminogen is present in the cytoplasm and nucleus. Plasminogen and LC3B (red fluorescence) co-localize in the cytoplasm (as shown by Δ), and the expression level of LC3B in the spinal cord tissue in the drug administration group seems to be less than that in the vehicle group. This shows that in amyotrophic lateral sclerosis model mice, plasminogen can enter the spinal cord tissue, enter the cell, and co-localize with LC3B, suggesting that there is an interaction between plasminogen and LC3B.

### Example 3 Plasminogen is enriched and co-localized with LAMP2 in the spinal cord tissue of the ALS model mice

Five wild-type male mice and nine male SOD1-G93A mice of similar age were taken. Wild-type mice were used as the blank control group. SOD1-G93A mice were observed and recorded from the 14th week when their hind legs trembled after the onset of the disease. The onset time of each mouse was recorded. Drug administration began 14 days after the onset of the disease. All mice were randomly divided into a vehicle group and a drug administration group according to the onset of the disease. Among them, there were 5 mice in the vehicle group, and 0.1 ml/mouse vehicle (sodium citrate buffer) was injected into the tail vein every day; there were 4 mice in the drug administration group, and 1 mg/0.1 ml/mouse plasminogen was injected into the tail vein every day. The drug administration was continuous under SPF environment, and the samples were collected at the end of death. The longest drug administration was 61 days. The spinal cord tissue was fixed in formalin fixing solution. The fixed tissue was subjected to gradient dehydration with alcohol and cleared with xylene before paraffin embedding. The thickness of the tissue section was 3 µm, and the sections were washed once after dewaxing and rehydration. The sections were immersed in the antigen retrieval working solution (0.01M sodium citrate buffer) and microwave repaired, preheating for 5 minutes, high heat for 2 minutes, and low heat for 15 minutes. The tissue was circled with a PAP pen, incubated with 3% hydrogen peroxide for 15 minutes, and washed twice with 0.01M PBS for 5 minutes each time. 5% of normal goat serum (Vector laboratories, Inc., USA) was used for blocking for 30 minutes. After the time was up, the goat serum was discarded, anti-plasminogen antibody (self-produced) was added dropwise, incubated overnight at 4°C, and washed twice with 0.01M PBS for 5 minutes each time. The goat anti-rabbit IgG (HRP) antibody (Abcam) as a secondary antibody was incubated at room temperature for 1 hour, and washed twice with 0.01M PBS for 5 minutes each time. According to the instructions of the XTSA520IHC kit (Alpha X Biotech, AXT6202500), the green fluorescence color development of the corresponding anti-plasminogen secondary antibody was performed. It was washed 3 times with PBS for 5 minutes each time. The above antigen retrieval and blocking operations were repeated, and then staining with anti-LAMP2 antibody (BOSTER, BM4357) was performed, incubating at 37°C for 1 hour. It was washed 3 times with PBS for 5 minutes each time. The goat anti-rabbit IgG (HRP) antibody (Abcam) as a secondary antibody was incubated at room temperature for 1 hour, and washed twice with 0.01M PBS for 5 minutes each time. According to the instructions of the XTSA620IHC kit (Alpha X Biotech, AXT6502500), the red fluorescence color development of the corresponding anti- LAMP2 secondary antibody was performed. It was washed 3 times with PBS for 5 minutes each time. Nuclear staining with DAPI (BOSTER, 11K16B77) was performed. It was washed 3 times with PBS for 5 minutes each time. The slice was dehydrated with gradient ethanol, cleared with xylene, sealed by neutral gum, observed and photographed under a 400x optical microscope.

Lysosomal associated membrane protein-2 (LAMP2) is a highly abundant lysosomal glycoprotein, which is a receptor for proteins imported directly into lysosomes and a mediator of autophagosome/phagosome maturation [4].

The results show that the positive staining of plasminogen (green fluorescence) in the spinal cord tissue in the drug administration group is significantly more than that in the vehicle group, indicating that the plasminogen can enter the spinal cord tissue and be enriched in the spinal cord tissue. In addition, plasminogen is present in the cytoplasm and nucleus. Plasminogen and LAMP2 (red fluorescence) co-localize in the cytoplasm (as shown by Δ), and the expression level of LAMP2 in the spinal cord tissue in the drug administration group seems to be more than that in the vehicle group (Figure 4). This shows that plasminogen can enter the cell, co-localize with LAMP2, and promote the expression of LAMP2, promoting the action of lysosome.

### Example 4 Plasminogen promotes the expression of LC3 in the spinal cord tissue of the amyotrophic lateral sclerosis model mice

Six SOD1-G93A mice aged 10-15 weeks were taken and randomly divided into two groups, three in the vehicle control group and three in the drug administration group. The mice in vehicle control group were injected with the vehicle by tail vein at 5 ml/kg, and the mice in the drug administration group were injected with plasminogen by tail vein (10 mg/ml) at 50 mg/kg of body weight. The mice were killed 24 hours after administration and the spinal cord was collected. After homogenization, RT-PCR detection of LC3 gene transcription was performed and the CT value was recorded. The CT value of mRNA transcribed by LC3 gene in 100 ng of total RNA was calculated.

The results show that the expression of LC3 in the drug administration group is significantly higher than that in the vehicle group, and the statistical difference is significant (* indicates P<0.05) (Figure 5). This indicates that plasminogen can promote the transcription of the LC3 gene in the amyotrophic lateral sclerosis model mice, thereby participating in the regulation of the autophagy process.

### Example 5 Plasminogen is enriched and co-localized with ubiquitin in the substantia nigra and striatum tissues of the Parkinson's model mice

Twelve C57 male mice aged 9 weeks were taken and weighed 1 day before modeling. The mice were intraperitoneally injected with 5 mg/ml MPTP solution at 30 mg/kg body weight every day for 5 consecutive days to establish a Parkinson's model [5, 6]. Preparation of MPTP solution: 10 ml of deionized water was sucked with a syringe and added to 100 mg of MPTP powder (sigma, M0896) to prepare a 10 mg/ml stock solution. Then 1 ml of the stock solution was sucked into an ampoule and 1 ml of deionized water was added to the final concentration of 5 mg/ml. After modeling, the mice were randomly divided into two groups, 6 mice each in the vehicle PBS control group and plasminogen group, and drug administration began, which was recorded as day 1. The mice in the plasminogen group were injected with the plasminogen solution by tail vein at 1 mg/0.1 ml/mouse/day, and the same volume of PBS was administrated by tail vein in the vehicle PBS control group. Drug administration continued for 14 days. The mice were killed on the day 15 of administration, and the substantia nigra and striatum of the mice were fixed in 4% paraformaldehyde for 24-48 hours. The fixed tissues were subjected to gradient dehydration with alcohol and cleared with xylene before paraffin embedding. Plasminogen, ubiquitin and DAPI were co-stained as described in Example 1. The sections were observed and photographed under a 400x optical microscope.

The results show that the positive staining of plasminogen (green fluorescence) in the substantia nigra and striatum tissues in the drug administration group is significantly more than that in the vehicle group, indicating that plasminogen can enter the substantia nigra and striatum tissues and be enriched in the substantia nigra and striatum tissues. In addition, plasminogen is present in the cytoplasm and nucleus. Plasminogen and ubiquitin (red fluorescence) co-localize in the cytoplasm (as shown by Δ) (Figure 6). This shows that in the Parkinson's model mice, plasminogen can enter the substantia nigra and striatum tissues, enter the cells, and co-localize with ubiquitin. This suggests that plasminogen may interact with ubiquitin.

### Example 6 Plasminogen is enriched and co-localized with LC3B in the substantia nigra and striatum of the Parkinson's model mice

Twelve C57 male mice aged 9 weeks were taken and weighed 1 day before modeling. The mice were intraperitoneally injected with 5 mg/ml MPTP solution at 30 mg/kg body weight every day for 5 consecutive days to establish a Parkinson's model [5, 6]. Preparation of MPTP solution: 10 ml of deionized water was sucked with a syringe and added to 100 mg of MPTP powder (sigma, M0896) to prepare a 10 mg/ml stock solution. Then 1 ml of the stock solution was sucked into an ampoule and 1 ml of deionized water was added to the final concentration of 5 mg/ml. After modeling, the mice were randomly divided into two groups, 6 mice each in the vehicle PBS control group and plasminogen group, and drug administration began, which was recorded as day 1. The mice in the plasminogen group were injected with the plasminogen solution by tail vein at 1 mg/0.1 ml/mouse/day, and the same volume of PBS was administrated by tail vein in the vehicle PBS control group. Drug administration continued for 14 days. The mice were killed on the day 15 of administration, and the substantia nigra and striatum of the mice were fixed in 4% paraformaldehyde for 24-48 hours. Subsequently, plasminogen, LC3B and DAPI were co-stained as described in Example 2. The sections were observed and photographed under a 400x optical microscope.

The results show that the positive staining of plasminogen (green fluorescence) in the substantia nigra and striatum tissues in the drug administration group is significantly more than that in the vehicle group, indicating that plasminogen can enter the substantia nigra and striatum tissues and be enriched in the substantia nigra and striatum tissues. In addition, plasminogen is present in the cytoplasm and nucleus. Plasminogen and LC3B (red fluorescence) co-localize in the cytoplasm (as shown by Δ) (Figure 7). This shows that plasminogen can enter the substantia nigra and striatum tissues, enter the cells, and co-localize with LC3B in the Parkinson's model mice. This suggests that plasminogen may interact with LC3.

### Example 7 Plasminogen is enriched and co-localized with LAMP2 in the substantia nigra and striatum tissues of the Parkinson's model mice

Twelve C57 male mice aged 9 weeks were taken and weighed 1 day before modeling. The mice were intraperitoneally injected with 5 mg/ml MPTP solution at 30 mg/kg body weight every day for 5 consecutive days to establish a Parkinson's model [5, 6]. Preparation of MPTP solution: 10 ml of deionized water was sucked with a syringe and added to 100 mg of MPTP powder (sigma, M0896) to prepare a 10 mg/ml stock solution. Then 1 ml of the stock solution was sucked into an ampoule and 1 ml of deionized water was added to the final concentration of 5 mg/ml. After modeling, the mice were randomly divided into two groups, 6 mice each in the vehicle PBS control group and plasminogen group, and drug administration began, which was recorded as day 1. The mice in the plasminogen group were injected with the plasminogen solution by tail vein at 1 mg/0.1 ml/mouse/day, and the same volume of PBS was administrated by tail vein in the vehicle PBS control group. Drug administration continued for 14 days. The mice were killed on the day 15 of administration, and the substantia nigra and striatum of the mice were fixed in 4% paraformaldehyde for 24-48 hours. Subsequently, plasminogen, LAMP2 and DAPI were co-stained as described in Example 3. The sections were observed and photographed under a 400x optical microscope.

The results show that the positive staining of plasminogen (green fluorescence) in the substantia nigra and striatum tissues in the drug administration group is significantly more than that in the vehicle group, indicating that the plasminogen can enter the substantia nigra and striatum tissues and be enriched in the substantia nigra and striatum tissues. In addition, plasminogen is present in the cytoplasm and nucleus. Plasminogen and LAMP2 (red fluorescence) co-localize in the cytoplasm (as shown by Δ) (Figure 8). It shows that in the Parkinson's model mice, plasminogen can enter the substantia nigra and striatum tissues, enter the cells, and co-localize with LAMP2. It suggests that plasminogen may interact with LAMP2.

### Example 8 Plasminogen is enriched and co-localized with ubiquitin in the hippocampal issues of the Alzheimer's model mice

Twenty C57 male mice aged 8 weeks were taken and weighed before modeling. After excluding abnormal mice according to their weight, all mice were randomly divided into two groups, a vehicle group and a drug administration group, with 10 mice in each group. All mice were anesthetized, and the granule cell layer of the hippocampus was located according to the mouse stereotaxic coordinates (based on the coordinate locations of the bregma: AP-2.0mm, ML±1.5mm, DV2.0mm). Each mouse was slowly microinjected bilaterally at an injection rate of 0.5 µL/min and an injection volume of 3 µL. The mice in the model group were injected with an Aβ1-42 oligomer solution to establish the Alzheimer's model [7], and the mice in the model control group were injected with PBS solution. Preparation of the Aβ1-42 oligomer solution (10 µM): β-Amyloid (1-42) (Shanghai Qiangyao Biotechnology Co., Ltd., 04010011521) was taken, and cold hexafluoroisopropanol was added to prepare a concentration of 1 mg/ml. It was placed at room temperature for 3 days, then divided into 45 µL/tube, i.e., 10 nmol/mL, placed in a fume hood overnight, placed in a 25°C drying oven for 1 hour, and stored at -80°C. When used, 10 µl of dimethyl sulfoxide solution was added to each tube for redissolution, and 990 µL of sterile PBS solution was added for injection and the solution was placed at 4°C for 24 hours before use. 21 days after the brain localization injection, the mice in the vehicle group and the drug administration group began to be administered, which was recorded as day 1. The mice in the drug administration group were injected with plasminogen by tail vein at 1 mg/0.1 ml/mouse/day, and the mice in the vehicle group were injected with 0.1 ml/mouse/day vehicle (4% arginine + 2% glycine solution) by tail vein, and the drug was administered continuously for 28 days. On day 29, the mice were killed and the brains were fixed in 10% formaldehyde for 24-48 hours. The fixed tissues were subjected to gradient dehydration with alcohol and cleared with xylene before paraffin embedding. Plasminogen, ubiquitin and DAPI were co-stained as described in Example 1. The sections were observed and photographed under a 400x optical microscope.

The results show that the positive staining of plasminogen (green fluorescence) in the hippocampal issues in the drug administration group is significantly more than that in the vehicle group, indicating that the plasminogen can enter the hippocampal issues and be enriched in the hippocampal issues. In addition, plasminogen is present in the cytoplasm and nucleus. Plasminogen and ubiquitin (red fluorescence) co-localize in the cytoplasm (as shown by Δ) (Figure 9). This indicates that plasminogen can enter the hippocampal issues and enter the cells in the Alzheimer's model mice. This suggests that plasminogen may interact with ubiquitin.

### Example 9 Plasminogen is enriched and co-localized with LC3B in the hippocampal issues of the Alzheimer's model mice

Twenty C57 male mice aged 8 weeks were taken and weighed before modeling. After excluding abnormal mice according to their weight, all mice were randomly divided into two groups, a vehicle group and a drug administration group, with 10 mice in each group. All mice were anesthetized, and the granule cell layer of the hippocampus was located according to the mouse stereotaxic coordinates (based on the coordinate locations of the bregma: AP-2.0mm, ML±1.5mm, DV2.0mm). Each mouse was slowly microinjected bilaterally at an injection rate of 0.5 µL/min and an injection volume of 3 µL. The mice in the model group were injected with an Aβ1-42 oligomer solution to establish the Alzheimer's model [7], and the mice in the model control group were injected with PBS solution. Preparation of the Aβ1-42 oligomer solution (10 µM): β-Amyloid (1-42) (Shanghai Qiangyao Biotechnology Co., Ltd., 04010011521) was taken, and cold hexafluoroisopropanol was added to prepare a concentration of 1 mg/ml. It was placed at room temperature for 3 days, then divided into 45 µL/tube, i.e., 10 nmol/mL, placed in a fume hood overnight, placed in a 25°C drying oven for 1 hour, and stored at -80°C. When used, 10 µl of dimethyl sulfoxide solution was added to each tube for redissolution, and 990 µL of sterile PBS solution was added for injection and the solution was placed at 4°C for 24 hours before use. 21 days after the brain localization injection, the mice in the vehicle group and the drug administration group began to be administered, which was recorded as day 1. The mice in the drug administration group were injected with plasminogen by tail vein at 1 mg/0.1 ml/mouse/day, and the mice in the vehicle group were injected with 0.1 ml/mouse/day vehicle (4% arginine + 2% glycine solution) by tail vein, and the drug was administered continuously for 28 days. On day 29, the mice were killed and the brains were fixed in 10% formaldehyde for 24-48 hours. The fixed tissues were subjected to gradient dehydration with alcohol and cleared with xylene before paraffin embedding. Plasminogen, LC3B and DAPI were co-stained as described in Example 2. The sections were observed and photographed under a 400x optical microscope.

The results show that the positive staining of plasminogen (green fluorescence) in the hippocampal issues in the drug administration group is significantly more than that in the vehicle group, indicating that the plasminogen can enter the hippocampal issues and be enriched in the hippocampal issues. In addition, plasminogen is present in the cytoplasm and nucleus. Plasminogen and LC3B (red fluorescence) co-localize in the cytoplasm (as shown by Δ) (Figure 10). This indicates that plasminogen can enter the hippocampal issues and enter the cells in the Alzheimer's model mice. This suggests that plasminogen may interact with LC3B.

### Example 10 Plasminogen is enriched and co-localized with LAMP2 in the hippocampal issues of the Alzheimer's model mice

Twenty C57 male mice aged 8 weeks were taken and weighed before modeling. After excluding abnormal mice according to their weight, all mice were randomly divided into two groups, a vehicle group and a drug administration group, with 10 mice in each group. All mice were anesthetized, and the granule cell layer of the hippocampal issues was located according to the mouse stereotaxic coordinates (based on the coordinate locations of the bregma: AP-2.0mm, ML±1.5mm, DV2.0mm). Each mouse was slowly microinjected bilaterally at an injection rate of 0.5 µL/min and an injection volume of 3 µL. The mice in the model group were injected with an Aβ1-42 oligomer solution to establish the Alzheimer's model [7], and the mice in the model control group were injected with PBS solution. Preparation of the Aβ1-42 oligomer solution (10 µM): β-Amyloid (1-42) (Shanghai Qiangyao Biotechnology Co., Ltd., 04010011521) was taken, and cold hexafluoroisopropanol was added to prepare a concentration of 1 mg/ml. It was placed at room temperature for 3 days, then divided into 45 µL/tube, i.e., 10 nmol/mL, placed in a fume hood overnight, placed in a 25°C drying oven for 1 hour, and stored at -80°C. When used, 10 µl of dimethyl sulfoxide solution was added to each tube for redissolution, and 990 µL of sterile PBS solution was added for injection and the solution was placed at 4°C for 24 hours before use. 21 days after the brain localization injection, the mice in the vehicle group and the drug administration group began to be administered, which was recorded as day 1. The mice in the drug administration group were injected with plasminogen by tail vein at 1 mg/0.1 ml/mouse/day, and the mice in the vehicle group were injected with 0.1 ml/mouse/day vehicle (4% arginine + 2% glycine solution) by tail vein, and the drug was administered continuously for 28 days. On day 29, the mice were killed and the brains were fixed in 10% formaldehyde for 24-48 hours. The fixed tissues were subjected to gradient dehydration with alcohol and cleared with xylene before paraffin embedding. Plasminogen, LAMP2 and DAPI were co-stained as described in Example 3. The sections were observed and photographed under a 400x optical microscope.

The results show that the positive staining of plasminogen (green fluorescence) in the hippocampal issues in the drug administration group is significantly more than that in the vehicle group, indicating that the plasminogen can enter the hippocampal issues and be enriched in the hippocampal issues. In addition, plasminogen is present in the cytoplasm and nucleus. Plasminogen and LAMP2 (red fluorescence) co-localize in the cytoplasm (as shown by Δ) (Figure 11). This indicates that plasminogen can enter the hippocampal issues and enter the cells in the Alzheimer's model mice. This suggests that plasminogen may interact with LAMP2.

### Example 11 Plasminogen promotes the degradation of huntingtin in the heart of the Huntington's model mice

Ten B6/JGpt-Tg(hHTT-CAG130)90/Gpt mice aged 14 weeks (referred to as hHTT130 transgenic mice) were selected as the model group, and five C57 mice were selected as the normal control group. The hHTT130 mice were randomly divided into two groups, the vehicle group and the drug administration group, with five mice in each group. After the grouping was completed, all mice began to be administered. The mice in the drug administration group were injected with 50 mg/kg plasminogen by tail vein, and the mice in the normal control group and the vehicle group were injected with the same volume of the vehicle by tail vein. The drug was administered continuously for 28 days. After drug administration, the mice were killed and the heart was taken. The heart tissue homogenate was used for huntingtin (HTT) detection by weastern blot. A 10% gel was prepared according to the SDS-PAGE dispensing instructions. The samples of each group were mixed with 4× loading buffer (TaKaRa, e2139) evenly at a volume ratio of 3:1, heated at 100°C for 5 min, cooled and centrifuged for 2min, and then 20µL was taken for loading. The electrophoresis conditions were 30V for 45 min, and then 100V for electrophoresis to the bottom of the gel. After the electrophoresis, the gel was stripped and transferred to an activated PVDF membrane (GE, A29433753), and the electrophoresis conditions were 15V for 2.5 h. The transferred PVDF membrane was immersed in a blocking solution (5% skim emulsion) and blocked overnight in a refrigerator at 4°C. After washing 4 times with TBST (0.01M Tris-NaCl, pH7.6 buffer), rabbit anti-HTT antibody (Abcam, ab109115) and actin antibody were added and incubated at room temperature for 1.5 h. After washing 4 times with TBST, goat anti-rabbit IgG (HRP) antibody (Abcam, ab6721) as a secondary antibody was added and incubated at room temperature for 1 h. After washing 4 times with TBST, the PVDF membrane was placed on a clean imaging plate, and Immobilon Western HRP Substrate (MILLIPORE, WBKLS0100) was added for color development. The membrane was photographed under a biomolecular imaging instrument and quantitatively analyzed using Image J.

The results show that the HTT level in the heart of the mice in the vehicle group is significantly higher than that of the mice in the normal control group, and the HTT level in the heart of the mice in the drug administration group is significantly lower than that of the mice in the vehicle group (Figure 12A-B), and the statistical difference is significant (* represents P<0.05). This suggests that plasminogen can promote the degradation of huntingtin in the heart tissue of the Huntington model mice.

### Example 12 Plasminogen promotes TDP-43 degradation in the cytoplasm and nucleus of NSC34 cells treated with okadaic acid

10⁶ NSC34 cells (Otwo Biotech, HTX1846) were inoculated in a 9cm² culture dish and cultured in DMEM medium (Gibco, 11965092) containing 10% fetal bovine serum (EVERY GREEN, 11011-8611), and placed in a carbon dioxide incubator for culture at 37.0°C and 5% CO₂. After the cells grew for 48 hours and the cells grew to about 80%-90% abundance, the medium was changed and subsequent experiments were performed. The cells were divided into 4 groups: blank control group, vehicle group, drug administration group and drug + EACA administration group. The cells in the blank control group were not treated after the medium was changed; the cells in the vehicle group, drug administration group and drug + EACA administration group were exposed to okadaic acid (OA) (Shanghai yuanye Bio-Technology, S30686-25ug) at a concentration of 2.5 ng/µL. After 24 hours of okadaic acid stimulation, the vehicle was added to the cell culture medium of the vehicle group, plasminogen (0.5 mg/mL) was added to the cell culture medium of the drug administration group, and plasminogen (final concentration was 0.5 mg/mL) and aminocaproic acid (20 mM) were added to the cell culture medium of the drug + EACA administration group. After adding plasminogen for 24 hours, the cells were harvested. The culture supernatant was sucked, washed with 1×PBS, digested with 1 mL of 0.25 pancreatin for 2-3 minutes, and the cells were obviously detached. The digestion was terminated with 5-6 mL of DMEM complete medium, and the cells were slowly blown. The suspension was collected into a centrifuge tube, and centrifuged at 1500 rpm for 5 min to remove the supernatant. The cells were resuspended with pre-cooled 1×PBS to count the cells. 200 µL of plasma protein extraction reagent was added to each 20 µL cell pellet (the volume of 2×10⁶ cell pellets is about 20 µL or 40 mg) (Solarbio, R0050). The cells were blown using a pipette or vortexed at high speed for 15 seconds to make the cell pellet completely dispersed into a single cell suspension. The cells were placed in the ice bath for 10 minutes, vortexed vigorously at the highest speed for 10 seconds, and centrifuged at 4°C, 12000-16000g for 10 minutes. The supernatant was the extracted cytoplasmic protein, and the supernatant was immediately sucked into a pre-cooled sample tube for use. The precipitate was the nucleus, and the remaining supernatant must be completely sucked (to avoid contamination of cytoplasmic protein), and 50-100 µL nuclear protein extraction reagent was added. The cell pellets were blown using a pipette or vortexed at high speed for 15 seconds (can be extended appropriately) to make the pellet completely dispersed, and placed in the ice bath for 10 minutes. The cell pellets were vortexed vigorously at the highest speed for 10 seconds, and centrifuged at 4°C, 12000-16000g for 10 minutes. The supernatant was immediately sucked into a pre-cooled sample tube, and the supernatant was the extracted cell nuclear protein. The extracted nuclear protein was detected by the TDP-43 western blot.

Aminocaproic acid (EACA) is a lysine analog that blocks the high-affinity lysine binding site on plasminogen [8].

The results show that the levels of TDP-43 in the cytoplasm and nucleus in the drug administration group are significantly lower than those in the nucleus of the vehicle group, and the addition of EACA can completely inhibit the effect of plasminogen on TDP-43 (Figure 13A-D). This suggests that plasminogen can promote the degradation of TDP-43 in the cytoplasm and nucleus, and this effect of plasminogen is closely related to the lysine binding site in its structure.

### Example 13 Plasminogen promotes TDP-43 degradation in the cytoplasm and nucleus of NSC34 cells treated with okadaic acid

10⁶ NSC34 cells (Otwo Biotech, HTX1846) were inoculated in a 9cm² culture dish and cultured in DMEM medium (Gibco, 11965092) containing 10% fetal bovine serum (EVERY GREEN, 11011-8611), and placed in a carbon dioxide incubator for culture at 37.0°C and 5% CO₂. After the cells grew for 48 hours and the cells grew to about 80%-90% abundance, the medium was changed and subsequent experiments were performed. The cells were divided into 4 groups: blank control group, vehicle group, drug administration group and drug + EACA administration group. The cells in the blank control group were not treated after the medium was changed; the cells in the vehicle group, drug administration group and drug + EACA administration group were exposed to okadaic acid (OA) (Shanghai yuanye Bio-Technology, S30686-25ug) at a concentration of 2.5 ng/µL. After 24 hours of okadaic acid stimulation, the vehicle was added to the cell culture medium of the vehicle group, plasminogen (0.5 mg/mL) was added to the cell culture medium of the drug administration group, and plasminogen (final concentration was 0.5 mg/mL) and aminocaproic acid (20 mM) were added to the cell culture medium of the drug + EACA administration group. After adding plasminogen for 24 hours, the cells were harvested. The culture supernatant was sucked, washed with 1×PBS, digested with 1 mL of 0.25 pancreatin for 2-3 minutes, and the cells were obviously detached. The digestion was terminated with 5-6 mL of DMEM complete medium, and the cells were slowly blown. The suspension was collected into a centrifuge tube, and centrifuged at 1500 rpm for 5 min to remove the supernatant. The cells were resuspended with pre-cooled 1×PBS to count the cells. Cell lysis buffer (Solarbio, R0010-100 mL) was added to lyse cells and extract cell proteins. The extracted cellular protein was detected by the TDP-43 western blot for phosphorylated Tau protein (Abcam, ab151559) and total Tau protein (Proteintech, 10842-1-AP).

The results show that the level of the phosphorylated Tau protein in the drug administration group is significantly lower than that in the vehicle group, and the addition of EACA can completely inhibit the effect of plasminogen on the phosphorylated Tau protein (Figure 14A-B); there is no significant difference between the total Tau protein level in the drug administration group and the vehicle group; the ratio of phosphorylated Tau protein to total Tau protein in the drug administration group is significantly lower than that in the vehicle group, and the addition of EACA can completely inhibit this effect of plasminogen (Figure 14C-E). * represents P<0.05, *** represents P<0.001. It suggests that plasminogen can promote the degradation of phosphorylated Tau protein in cells, and this effect of plasminogen is closely related to the lysine binding site in its structure.

### Example 14 Plasminogen promotes the increase of plasminogen level and plasmin activity level in the cytoplasm and nucleus of NSC34 cells treated with okadaic acid

10⁶ NSC34 cells (Otwo Biotech, HTX1846) were inoculated in a 9cm² culture dish and cultured in DMEM medium (Gibco, 11965092) containing 10% fetal bovine serum (EVERY GREEN, 11011-8611), and placed in a carbon dioxide incubator for culture at 37.0°C and 5% CO₂. After the cells grew for 48 hours and the cells grew to about 80%-90% abundance, the medium was changed and subsequent experiments were performed. The cells were divided into 3 groups: vehicle group, drug administration group and drug + EACA administration group. The cells in the vehicle group, drug administration group and drug + EACA administration group were exposed to okadaic acid (OA) (Shanghai yuanye Bio-Technology, S30686-25ug) at a concentration of 2.5 ng/µL. After 24 hours of okadaic acid stimulation, the vehicle was added to the cell culture medium of the vehicle group, plasminogen (0.5 mg/mL) was added to the cell culture medium of the drug administration group, and plasminogen (final concentration was 0.5 mg/mL) and aminocaproic acid (20 mM) were added to the cell culture medium of the drug + EACA administration group. After adding plasminogen for 24 hours, the cells were harvested. The culture supernatant was sucked, washed with 1×PBS, digested with 1 mL of 0.25 pancreatin for 2-3 minutes, and the cells were obviously detached. The digestion was terminated with 5-6 mL of DMEM complete medium, and the cells were slowly blown. The suspension was collected into a centrifuge tube, and centrifuged at 1500 rpm for 5 min to remove the supernatant. The cells were resuspended with pre-cooled 1×PBS to count the cells. 200 µL of plasma protein extraction reagent was added to each 20 µL cell pellet (the volume of 2×10⁶ cell pellets is about 20 µL or 40 mg) (Solarbio, R0050). The cell pellets were blown using a pipette or vortexed at high speed for 15 seconds to make the cell pellet completely dispersed into a single cell suspension. The cells were placed in the ice bath for 10 minutes, vortexed vigorously at the highest speed for 10 seconds, and centrifuged at 4°C, 12000-16000g for 10 minutes. The supernatant was the extracted cytoplasmic protein, and the supernatant was immediately sucked into a pre-cooled sample tube for use. The precipitate was the nucleus, and the remaining supernatant must be completely sucked (to avoid contamination of cytoplasmic protein), and 50-100 µL nuclear protein extraction reagent was added. The cell pellets were blown using a pipette or vortexed at high speed for 15 seconds (can be extended appropriately) to make the pellet completely dispersed, and placed in the ice bath for 10 minutes. The cell pellets were vortexed vigorously at the highest speed for 10 seconds, and centrifuged at 4°C, 12000-16000g for 10 minutes. The supernatant was immediately sucked into a pre-cooled sample tube, and the supernatant was the extracted cell nuclear protein.

After cell lysis, the detection was performed according to the instructions of Human Plasminogen ELISA Kit (Manufacturer: AssayMax, Catalog No.: EP1200-1). The human plasminogen working standard in the kit was used as the internal standard to calibrate the concentration of each sample. The calibrated concentration was divided by the total protein concentration to calculate the amount of plasminogen per unit of total protein in each sample, and statistical analysis was performed.

The activity of plasmin was detected by the enzyme substrate kinetic method. 85 µL/well of the standard solution at seven different concentrations, blank, and sample were added to the ELISA plate in turn, and then 15 µL of 20 mM S-2251 solution (Chromogenix, 82033239) was added to each well and incubated at 37°C. Starting from 0 min of reaction, the A405 absorbance value was read in a multifunctional microplate reader every 5 min until 90 min of reaction. All reactions were linearly fitted with time and absorbance, and the slope of the straight line was the reaction rate of the standard/sample (△A405/min). Finally, the titer value of the standard and △A405/min were used to make the standard curve to calculate the titer of the test sample.

The results show that the levels of human plasminogen and plasmin activity in the cytoplasm and nucleus in the drug administration group is significantly higher than that in the vehicle group, and the statistical difference is significant; and the addition of EACA can completely inhibit the effects of plasminogen (Figure 15A-D). It suggests that plasminogen can enter cells and even nucleus to increase plasmin activity, and the entry of plasminogen into cells and nucleus is closely related to its lysine binding activity.

### Example 15 Plasminogen promotes the degradation of huntingtin in the kidney of the Huntington's model mice

Ten B6/JGpt-Tg (hHTT-CAG130) 90/Gpt mice aged 14 weeks (referred to as hHTT130 transgenic mice) were selected as the model group, and five C57 mice were selected as the normal control group. The hHTT130 mice were randomly divided into two groups, the vehicle group and the drug administration group, with five mice in each group. After the grouping was completed, all mice began to be administered. The mice in the drug administration group were injected with 50 mg/kg plasminogen by tail vein, and the mice in the normal control group and the vehicle group were injected with the same volume of the vehicle by tail vein. The drug was administered continuously for 28 days. After drug administration, the mice were killed and the kidney was taken. The kidney tissue homogenate was used for huntingtin (HTT) detection by weastern blot. A 10% gel was prepared according to the SDS-PAGE dispensing instructions. The samples of each group were mixed with 4× loading buffer (TaKaRa, e2139) evenly at a volume ratio of 3:1, heated at 100°C for 5 min, cooled and centrifuged for 2min, and then 20µL was taken for loading. The electrophoresis conditions were 30V for 45 min, and then 100V for electrophoresis to the bottom of the gel. After the electrophoresis, the gel was stripped and transferred to an activated PVDF membrane (GE, A29433753), and the electrophoresis conditions were 15V for 2.5 h. The transferred PVDF membrane was immersed in a blocking solution (5% skim emulsion) and blocked overnight in a refrigerator at 4°C. After washing 4 times with TBST (0.01M Tris-NaCl, pH7.6 buffer), rabbit anti-HTT antibody (Abcam, ab109115) and actin antibody were added and incubated at room temperature for 1.5 h. After washing 4 times with TBST, goat anti-rabbit IgG (HRP) antibody (Abcam, ab6721) as a secondary antibody was added and incubated at room temperature for 1 h. After washing 4 times with TBST, the PVDF membrane was placed on a clean imaging plate, and Immobilon Western HRP Substrate (MILLIPORE, WBKLS0100) was added for color development. The membrane was photographed under a biomolecular imaging instrument and quantitatively analyzed using Image J.

The results show that the HTT level in the kidney of the mice in the vehicle group is significantly higher than that of the mice in the normal control group, and the HTT level in the kidney of the mice of the drug administration group is significantly lower than that of the mice in the vehicle group (Figure 16A-B), and the statistical difference is significant (* represents P<0.05). This suggests that plasminogen can promote the degradation of huntingtin in the kidney tissue of the Huntington model mice.

### Example 16 Plasminogen promotes the degradation of huntingtin in brain tissue of Huntington model mice

B6/JGpt-Tg(hHTT-CAG130)90/Gpt mice aged 2 weeks were selected and killed, and brain tissues were taken and placed in pre-cooled 1640 culture medium (manufacturer: Gibco, catalog number: 31800-105). A cellulose acetate filter membrane with the pore size of 0.8 µm was placed at the bottom of a 6-well plate, 1 mL of B27 culture medium (manufacturer: Gibco, catalog number: A3653401) was added to the filter membrane to wet the filter membrane. The brain tissue was placed in a flat dish containing pre-cooled PBS, rinsed 3 times with 1×PBS, cleaned to remove the blood on the surface of the tissue, and cut into thin slices using a blade. The brain slices were divided into 6 samples, laid out on the filter membrane with culture medium to ensure that the culture medium can just immerse the brain slices, and cultured. The 6-well plate was placed in a CO₂ incubator, and were cultured at 37°C, 5% CO₂. On the second day, the culture medium was changed, 0.4 mg/mL plasminogen was added to the drug administration group (3 samples), and the same volume of saline was added to the vehicle group (3 samples) at the same time. The culture medium was changed every day, and the corresponding experimental group was added with the same amount of plasminogen or saline as above. After 3 days of culture (plasminogen acted for 2 days), the brain slices and culture medium were sucked, centrifuged at 1200 rpm for 5 min, and the supernatant was removed. Then the brain slices were resuspended with 1×PBS, and centrifuged again to remove PBS. The brain tissue precipitate was preserved. After homogenization, huntingtin (HTT) was detected by weastern blot. A 10% gel was prepared according to the SDS-PAGE dispensing instructions. The samples of each group were mixed with 4× loading buffer (TaKaRa, e2139) evenly at a volume ratio of 3:1, heated at 100°C for 5 min, cooled and centrifuged for 2min, and then 20µL was taken for loading. The electrophoresis conditions were 30V for 45 min, and then 100V for electrophoresis to the bottom of the gel. After the electrophoresis, the gel was stripped and transferred to an activated PVDF membrane (GE, A29433753), and the electrophoresis conditions were 15V for 2.5 h. The transferred PVDF membrane was immersed in a blocking solution (5% skim emulsion) and blocked overnight in a refrigerator at 4°C. After washing 4 times with TBST (0.01M Tris-NaCl, pH7.6 buffer), rabbit anti-HTT antibody (Abcam, ab109115) and actin antibody were added and incubated at room temperature for 1.5 h. After washing 4 times with TBST, goat anti-rabbit IgG (HRP) antibody (Abcam, ab6721) as a secondary antibody was added and incubated at room temperature for 1 h. After washing 4 times with TBST, the PVDF membrane was placed on a clean imaging plate, and Immobilon Western HRP Substrate (MILLIPORE, WBKLS0100) was added for color development. The membrane was photographed under a biomolecular imaging instrument and quantitatively analyzed using Image J.

The results show that the HTT level in the brain tissue of the mice in the drug administration group is significantly lower than that of the mice in the vehicle group, and the statistical difference is significant (* represents P<0.05). This suggests that plasminogen can promote the degradation of huntingtin in the brain tissue of the Huntington model mice.

### References

[1]KENNETH C. ROBBINS, LOUIS SUMMARIA, DAVID ELWYN et al. Further Studies on the Purification and Characterization of Human Plasminogen and Plasmin. Journal of Biological Chemistry , 1965 , 240 (1) :541-550.
[2]Summaria L, Spitz F, Arzadon L et al. Isolation and characterization of the affinity chromatography forms of human Glu- and Lys-plasminogens and plasmins. J Biol Chem. 1976 Jun 25;251(12):3693-9.
[3]HAGAN JJ, ABLONDI FB, DE RENZO EC. Purification and biochemical properties of human plasminogen. J Biol Chem. 1960 Apr;235:1005-10.
[4] Eskelinen EL. Roles of LAMP-1 and LAMP-2 in lysosome biogenesis and autophagy. Mol Aspects Med. 2006 Oct-Dec;27(5-6):495-502
[5]Vernice Jackson-Lewis1 & Serge Przedborski. Protocol for the MPTP mouse model of Parkinson's Disease [J]. Nature protocols VOL.2 NO.1, 2007(141).
[6]N. A. Tatton and S. J. Kish. In situ detection of apoptotic nuclei in the substantia aigra compacta of 1-methyl-4-phenyl-1,2,3,6-tetrahydropyridine-treated mice using terminal deoxynucleotidyl transferase labeling and acridine orange staining[J].Neuroscience Vol.77, No.4,pp.103 7-1048, 1997.
[7]Moon M, Choi J G, Kim S Y, et al. Bombycis Excrementum Reduces Amyloid- β Oligomer-Induced Memory Impairments, Neurodegeneration, and Neuroinflammation in Mice[J]. Journal of Alzheimer's disease: JAD, 2014, 41(2).
[8] Sun Z, Chen YH, Wang P, Zhang J, Gurewich V, Zhang P, Liu JN. The blockage of the high-affinity lysine binding sites of plasminogen by EACA significantly inhibits prourokinase-induced plasminogen activation. Biochim Biophys Acta. 2002 Apr 29;1596(2):182-92.

### Sequence Listing

Sequence 1 (nucleic acid sequence of the natural plasminogen (Glu-PLG, Glu-plasminogen) without signal peptide): 
gagcctctggatgactatgtgaatacccaggggcttcactgttcagtgtcactaagaagcagctgggagcaggaagtatagaagaatgtgcagcaaaatgtgaggag
gacgaagaattcacctgcagggcattccaatatcacagtaaagagcaacaatgtgtgataatggctgaaaacaggaagtcctccataatcattaggatgagagatgtagt
tttatttgaaaagaaagtgtatctctcagagtgcaagactgggaatggaaagaactacagagggacgatgtccaaaacaaaaaatggcatcacctgtcaaaaatggagtt
ccacttctccccacagacctagattctcacctgctacacacccctcagagggactggaggagaactactgcaggaatccagacaacgatccgcaggggccctggtgct
atactactgatccagaaaagagatatgactactgcgacattcttgagtgtgaagaggaatgtatgcattgcagtggagaaaactatgacggcaaaatttccaagaccatgt
ctggactggaatgccaggcctgggactctcagagcccacacgctcatggatacattccttccaaatttccaaacaagaacctgaagaagaattactgtcgtaaccccgat
agggagctgcggccttggtgtttcaccaccgaccccaacaagcgctgggaactttgtgacatccccgctgcacaacacctccaccatcttctggtcccacctaccagt
gtctgaagggaacaggtgaaaactatcgcgggaatgtggctgttaccgtgtccgggcacacctgtcagcactggagtgcacagacccctcacacacataacaggaca
ccagaaaacttcccctgcaaaaatttggatgaaaactactgccgcaatcctgacggaaaaagggccccatggtgccatacaaccaacagccaagtgcggtgggagta
ctgtaagataccgtcctgtgactcctccccagtatccacggaacaattggctcccacagcaccacctgagctaacccctgtggtccaggactgctaccatggtgatggac
agagctaccgaggcacatcctccaccaccaccacaggaaagaagtgtcagtcttggtcatctatgacaccacaccggcaccagaagaccccagaaaactacccaaat
gctggcctgacaatgaactactgcaggaatccagatgccgataaaggcccctggtgttttaccacagaccccagcgtcaggtgggagtactgcaacctgaaaaaatgc
tcaggaacagaagcgagtgttgtagcacctccgcctgttgtcctgcttccagatgtagagactccttccgaagaagactgtatgtttgggaatgggaaaggataccgagg
caagagggcgaccactgttactgggacgccatgccaggactgggctgcccaggagccccatagacacagcattttcactccagagacaaatccacgggcgggtctg
gaaaaaaattactgccgtaaccctgatggtgatgtaggtggtccctggtgctacacgacaaatccaagaaaactttacgactactgtgatgtccctcagtgtgcggcccct
tcatttgattgtgggaagcctcaagtggagccgaagaaatgtcctggaagggttgtaggggggtgtgtggcccacccacattcctggccctggcaagtcagtcttagaa
caaggtttggaatgcacttctgtggaggcaccttgatatccccagagtgggtgttgactgctgcccactgcttggagaagtccccaaggccttcatcctacaaggtcatcc
tgggtgcacaccaagaagtgaatctcgaaccgcatgttcaggaaatagaagtgtctaggctgttcttggagcccacacgaaaagatattgccttgctaaagctaagcagt
cctgccgtcatcactgacaaagtaatcccagcttgtctgccatccccaaattatgtggtcgctgaccggaccgaatgtttcatcactggctggggagaaacccaaggtact
tttggagctggccttctcaaggaagcccagctccctgtgattgagaataaagtgtgcaatcgctatgagtttctgaatggaagagtccaatccaccgaactctgtgctggg
catttggccggaggcactgacagttgccagggtgacagtggaggtcctctggtttgcttcgagaaggacaaatacattttacaaggagtcacttcttggggtcttggctgt
gcacgccccaataagcctggtgtctatgttcgtgtttcaaggtttgttacttggattgagggagtgatgagaaataattaa
SEQ ID NO: 2 (amino acid sequence of the natural plasminogen (Glu-PLG, Glu-plasminogen) without signal peptide): 
EPLDDYVNTQGASLFSVTKKQLGAGSIEECAAKCEEDEEFTCRAFQYHSKEQQCVIMAENRKSSIIIRMRD
VVLFEKKVYLSECKTGNGKNYRGTMSKTKNGITCQKWSSTSPHRPRFSPATHPSEGLEENYCRNPDNDPQ
GPWCYTTDPEKRYDYCDILECEEECMHCSGENYDGKISKTMSGLECQAWDSQSPHAHGYIPSKFPNKNLK
KNYCRNPDRELRPWCFTTDPNKRWELCDIPRCTTPPPSSGPTYQCLKGTGENYRGNVAVTVSGHTCQHW
SAQTPHTHNRTPENFPCKNLDENYCRNPDGKRAPWCHTTNSQVRWEYCKIPSCDSSPVSTEQLAPTAPPEL
TPVVQDCYHGDGQSYRGTSSTTTTGKKCQSWSSMTPHRHQKTPENYPNAGLTMNYCRNPDADKGPWCF
TTDPSVRWEYCNLKKCSGTEASVVAPPPVVLLPDVETPSEEDCMFGNGKGYRGKRATTVTGTPCQDWAA
 
QEPHRHSIFTPETNPRAGLEKNYCRNPDGDVGGPWCYTTNPRKLYDYCDVPQCAAPSFDCGKPQVEPKKC
PGRVVGGCVAHPHSWPWQVSLRTRFGMHFCGGTLISPEWVLTAAHCLEKSPRPSSYKVILGAHQEVNLEP
HVQEIEVSRLFLEPTRKDIALLKLSSPAVITDKVIPACLPSPNYVVADRTECFITGWGETQGTFGAGLLKEA
QLPVIENKVCNRYEFLNGRVQSTELCAGHLAGGTDSCQGDSGGPLVCFEKDKYILQGVTSWGLGCARPN
KPGVYVRVSRFVTWIEGVMRNN
SEQ ID NO: 3 (nucleic acid sequence of the natural plasminogen (derived from swiss prot) with a signal peptide): 
atggaacataaggaagtggttcttctacttcttttatttctgaaatcaggtcaaggagagcctctggatgactatgtgaatacccaggggcttcactgttcagtgtcactaag
aagcagctgggagcaggaagtatagaagaatgtgcagcaaaatgtgaggaggacgaagaattcacctgcagggcattccaatatcacagtaaagagcaacaatgtgt
gataatggctgaaaacaggaagtcctccataatcattaggatgagagatgtagttttatttgaaaagaaagtgtatctctcagagtgcaagactgggaatggaaagaacta
cagagggacgatgtccaaaacaaaaaatggcatcacctgtcaaaaatggagttccacttctccccacagacctagattctcacctgctacacacccctcagagggactg
gaggagaactactgcaggaatccagacaacgatccgcaggggccctggtgctatactactgatccagaaaagagatatgactactgcgacattcttgagtgtgaagag
gaatgtatgcattgcagtggagaaaactatgacggcaaaatttccaagaccatgtctggactggaatgccaggcctgggactctcagagcccacacgctcatggataca
ttccttccaaatttccaaacaagaacctgaagaagaattactgtcgtaaccccgatagggagctgcggccttggtgtttcaccaccgaccccaacaagcgctgggaacttt
gtgacatcccccgctgcacaacacctccaccatcttctggtcccacctaccagtgtctgaagggaacaggtgaaaactatcgcgggaatgtggctgttaccgtgtccgg
gcacacctgtcagcactggagtgcacagacccctcacacacataacaggacaccagaaaacttcccctgcaaaaatttggatgaaaactactgccgcaatcctgacgg
aaaaagggccccatggtgccatacaaccaacagccaagtgcggtgggagtactgtaagataccgtcctgtgactcctccccagtatccacggaacaattggctcccac
agcaccacctgagctaacccctgtggtccaggactgctaccatggtgatggacagagctaccgaggcacatcctccaccaccaccacaggaaagaagtgtcagtcttg
gtcatctatgacaccacaccggcaccagaagaccccagaaaactacccaaatgctggcctgacaatgaactactgcaggaatccagatgccgataaaggcccctggt
gttttaccacagaccccagcgtcaggtgggagtactgcaacctgaaaaaatgctcaggaacagaagcgagtgttgtagcacctccgcctgttgtcctgcttccagatgta
gagactccttccgaagaagactgtatgtttgggaatgggaaaggataccgaggcaagagggcgaccactgttactgggacgccatgccaggactgggctgcccagg
agccccatagacacagcattttcactccagagacaaatccacgggcgggtctggaaaaaaattactgccgtaaccctgatggtgatgtaggtggtccctggtgctacac
gacaaatccaagaaaactttacgactactgtgatgtccctcagtgtgcggccccttcatttgattgtgggaagcctcaagtggagccgaagaaatgtcctggaagggttgt
aggggggtgtgtggcccacccacattcctggccctggcaagtcagtcttagaacaaggtttggaatgcacttctgtggaggcaccttgatatccccagagtgggtgttga
ctgctgcccactgcttggagaagtccccaaggccttcatcctacaaggtcatcctgggtgcacaccaagaagtgaatctcgaaccgcatgttcaggaaatagaagtgtct
aggctgttcttggagcccacacgaaaagatattgccttgctaaagctaagcagtcctgccgtcatcactgacaaagtaatcccagcttgtctgccatccccaaattatgtgg
tcgctgaccggaccgaatgtttcatcactggctggggagaaacccaaggtacttttggagctggccttctcaaggaagcccagctccctgtgattgagaataaagtgtgc
aatcgctatgagtttctgaatggaagagtccaatccaccgaactctgtgctgggcatttggccggaggcactgacagttgccagggtgacagtggaggtcctctggtttg
cttcgagaaggacaaatacattttacaaggagtcacttcttggggtcttggctgtgcacgccccaataagcctggtgtctatgttcgtgtttcaaggtttgttacttggattgag
ggagtgatgagaaataattaa
SEQ ID NO: 4 (amino acid sequence of the natural plasminogen (derived from swiss prot) with a signal peptide):
SEQ ID NO: 5 (nucleic acid sequence of LYS77-PLG (Lys-plasminogen)): 
aaagtgtatctctcagagtgcaagactgggaatggaaagaactacagagggacgatgtccaaaacaaaaaatggcatcacctgtcaaaaatggagttccacttctcccc
acagacctagattctcacctgctacacacccctcagagggactggaggagaactactgcaggaatccagacaacgatccgcaggggccctggtgctatactactgatc
cagaaaagagatatgactactgcgacattcttgagtgtgaagaggaatgtatgcattgcagtggagaaaactatgacggcaaaatttccaagaccatgtctggactggaa
tgccaggcctgggactctcagagcccacacgctcatggatacattccttccaaatttccaaacaagaacctgaagaagaattactgtcgtaaccccgatagggagctgc
ggccttggtgtttcaccaccgaccccaacaagcgctgggaactttgtgacatcccccgctgcacaacacctccaccatcttctggtcccacctaccagtgtctgaaggga
acaggtgaaaactatcgcgggaatgtggctgttaccgtgtccgggcacacctgtcagcactggagtgcacagacccctcacacacataacaggacaccagaaaacttc
ccctgcaaaaatttggatgaaaactactgccgcaatcctgacggaaaaagggccccatggtgccatacaaccaacagccaagtgcggtgggagtactgtaagatacc
gtcctgtgactcctccccagtatccacggaacaattggctcccacagcaccacctgagctaacccctgtggtccaggactgctaccatggtgatggacagagctaccga
ggcacatcctccaccaccaccacaggaaagaagtgtcagtcttggtcatctatgacaccacaccggcaccagaagaccccagaaaactacccaaatgctggcctgac
aatgaactactgcaggaatccagatgccgataaaggcccctggtgttttaccacagaccccagcgtcaggtgggagtactgcaacctgaaaaaatgctcaggaacaga
agcgagtgttgtagcacctccgcctgttgtcctgcttccagatgtagagactccttccgaagaagactgtatgtttgggaatgggaaaggataccgaggcaagagggcg
accactgttactgggacgccatgccaggactgggctgcccaggagccccatagacacagcattttcactccagagacaaatccacgggcgggtctggaaaaaaatta
ctgccgtaaccctgatggtgatgtaggtggtccctggtgctacacgacaaatccaagaaaactttacgactactgtgatgtccctcagtgtgcggccccttcatttgattgtg
ggaagcctcaagtggagccgaagaaatgtcctggaagggttgtaggggggtgtgtggcccacccacattcctggccctggcaagtcagtcttagaacaaggtttggaa
tgcacttctgtggaggcaccttgatatccccagagtgggtgttgactgctgcccactgcttggagaagtccccaaggccttcatcctacaaggtcatcctgggtgcacacc
aagaagtgaatctcgaaccgcatgttcaggaaatagaagtgtctaggctgttcttggagcccacacgaaaagatattgccttgctaaagctaagcagtcctgccgtcatca
ctgacaaagtaatcccagcttgtctgccatccccaaattatgtggtcgctgaccggaccgaatgtttcatcactggctggggagaaacccaaggtacttttggagctggcc
ttctcaaggaagcccagctccctgtgattgagaataaagtgtgcaatcgctatgagtttctgaatggaagagtccaatccaccgaactctgtgctgggcatttggccggag
gcactgacagttgccagggtgacagtggaggtcctctggtttgcttcgagaaggacaaatacattttacaaggagtcacttcttggggtcttggctgtgcacgccccaata
agcctggtgtctatgttcgtgtttcaaggtttgttacttggattgagggagtgatgagaaataattaa
SEQ ID NO: 6 (amino acid sequence of LYS77-PLG (Lys-plasminogen)): 
KVYLSECKTGNGKNYRGTMSKTKNGITCQKWSSTSPHRPRFSPATHPSEGLEENYCRNPDNDPQGPWCYT
TDPEKRYDYCDILECEEECMHCSGENYDGKISKTMSGLECQAWDSQSPHAHGYIPSKFPNKNLKKNYCRN
PDRELRPWCFTTDPNKRWELCDIPRCTTPPPSSGPTYQCLKGTGENYRGNVAVTVSGHTCQHWSAQTPHT
HNRTPENFPCKNLDENYCRNPDGKRAPWCHTTNSQVRWEYCKIPSCDSSPVSTEQLAPTAPPELTPVVQD
CYHGDGQSYRGTSSTTTTGKKCQSWSSMTPHRHQKTPENYPNAGLTMNYCRNPDADKGPWCFTTDPSV
RWEYCNLKKCSGTEASVVAPPPVVLLPDVETPSEEDCMFGNGKGYRGKRATTVTGTPCQDWAAQEPHR
HSIFTPETNPRAGLEKNYCRNPDGDVGGPWCYTTNPRKLYDYCDVPQCAAPSFDCGKPQVEPKKCPGRV
VGGCVAHPHSWPWQVSLRTRFGMHFCGGTLISPEWVLTAAHCLEKSPRPSSYKVILGAHQEVNLEPHVQE
IEVSRLFLEPTRKDIALLKLSSPAVITDKVIPACLPSPNYVVADRTECFITGWGETQGTFGAGLLKEAQLPVI
ENKVCNRYEFLNGRVQSTELCAGHLAGGTDSCQGDSGGPLVCFEKDKYILQGVTSWGLGCARPNKPGVY
VRVSRFVTWIEGVMRNN
SEQ ID NO: 7 (nucleic acid sequence of delta-plg (delta-plasminogen)): 
gagcctctggatgactatgtgaatacccagggggcttcactgttcagtgtcactaagaagcagctgggagcaggaagtatagaagaatgtgcagcaaaatgtgaggag
gacgaagaattcacctgcagggcattccaatatcacagtaaagagcaacaatgtgtgataatggctgaaaacaggaagtcctccataatcattaggatgagagatgtagt
tttatttgaaaagaaagtgtatctctcagagtgcaagactgggaatggaaagaactacagagggacgatgtccaaaacaaaaaatggcatcacctgtcaaaaatggagtt
ccacttctccccacagacctagattctcacctgctacacacccctcagagggactggaggagaactactgcaggaatccagacaacgatccgcaggggccctggtgct
atactactgatccagaaaagagatatgactactgcgacattcttgagtgtgaagaggcggccccttcatttgattgtgggaagcctcaagtggagccgaagaaatgtcct
ggaagggttgtaggggggtgtgtggcccacccacattcctggccctggcaagtcagtcttagaacaaggtttggaatgcacttctgtggaggcaccttgatatccccag
agtgggtgttgactgctgcccactgcttggagaagtccccaaggccttcatcctacaaggtcatcctgggtgcacaccaagaagtgaatctcgaaccgcatgttcagga
 
aatagaagtgtctaggctgttcttggagcccacacgaaaagatattgccttgctaaagctaagcagtcctgccgtcatcactgacaaagtaatcccagcttgtctgccatcc
ccaaattatgtggtcgctgaccggaccgaatgtttcatcactggctggggagaaacccaaggtacttttggagctggccttctcaaggaagcccagctccctgtgattga
gaataaagtgtgcaatcgctatgagtttctgaatggaagagtccaatccaccgaactctgtgctgggcatttggccggaggcactgacagttgccagggtgacagtgga
ggtcctctggtttgcttcgagaaggacaaatacattttacaaggagtcacttcttggggtcttggctgtgcacgccccaataagcctggtgtctatgttcgtgtttcaaggttt
gttacttggattgagggagtgatgagaaataattaa
SEQ ID NO: 8 (amino acid sequence of delta-plg (delta-plasminogen)): 
EPLDDYVNTQGASLFSVTKKQLGAGSIEECAAKCEEDEEFTCRAFQYHSKEQQCVIMAENRKSSIIIRMRD
VVLFEKKVYLSECKTGNGKNYRGTMSKTKNGITCQKWSSTSPHRPRFSPATHPSEGLEENYCRNPDNDPQ
GPWCYTTDPEKRYDYCDILECEEAAPSFDCGKPQVEPKKCPGRVVGGCVAHPHSWPWQVSLRTRFGMHF
CGGTLISPEWVLTAAHCLEKSPRPSSYKVILGAHQEVNLEPHVQEIEVSRLFLEPTRKDIALLKLSSPAVITD
KVIPACLPSPNYVVADRTECFITGWGETQGTFGAGLLKEAQLPVIENKVCNRYEFLNGRVQSTELCAGHL
AGGTDSCQGDSGGPLVCFEKDKYILQGVTSWGLGCARPNKPGVYVRVSRFVTWIEGVMRNN
SEQ ID NO: 9 (nucleic acid sequence of Mini-plg (mini-plasminogen)): 
gtcaggtgggagtactgcaacctgaaaaaatgctcaggaacagaagcgagtgttgtagcacctccgcctgttgtcctgcttccagatgtagagactccttccgaagaag
actgtatgtttgggaatgggaaaggataccgaggcaagagggcgaccactgttactgggacgccatgccaggactgggctgcccaggagccccatagacacagcat
tttcactccagagacaaatccacgggcgggtctggaaaaaaattactgccgtaaccctgatggtgatgtaggtggtccctggtgctacacgacaaatccaagaaaacttt
acgactactgtgatgtccctcagtgtgcggccccttcatttgattgtgggaagcctcaagtggagccgaagaaatgtcctggaagggttgtaggggggtgtgtggccca
cccacattcctggccctggcaagtcagtcttagaacaaggtttggaatgcacttctgtggaggcaccttgatatccccagagtgggtgttgactgctgcccactgcttgga
gaagtccccaaggccttcatcctacaaggtcatcctgggtgcacaccaagaagtgaatctcgaaccgcatgttcaggaaatagaagtgtctaggctgttcttggagccca
cacgaaaagatattgccttgctaaagctaagcagtcctgccgtcatcactgacaaagtaatcccagcttgtctgccatccccaaattatgtggtcgctgaccggaccgaat
gtttcatcactggctggggagaaacccaaggtacttttggagctggccttctcaaggaagcccagctccctgtgattgagaataaagtgtgcaatcgctatgagtttctgaa
tggaagagtccaatccaccgaactctgtgctgggcatttggccggaggcactgacagttgccagggtgacagtggaggtcctctggtttgcttcgagaaggacaaatac
attttacaaggagtcacttcttggggtcttggctgtgcacgccccaataagcctggtgtctatgttcgtgtttcaaggtttgttacttggattgagggagtgatgagaaataatt
aa
SEQ ID NO: 10 (amino acid sequence of Mini-plg (mini-plasminogen)): 
VRWEYCNLKKCSGTEASVVAPPPVVLLPDVETPSEEDCMFGNGKGYRGKRATTVTGTPCQDWAAQEPH
RHSIFTPETNPRAGLEKNYCRNPDGDVGGPWCYTTNPRKLYDYCDVPQCAAPSFDCGKPQVEPKKCPGR
VVGGCVAHPHSWPWQVSLRTRFGMHFCGGTLISPEWVLTAAHCLEKSPRPSSYKVILGAHQEVNLEPHV
QEIEVSRLFLEPTRKDIALLKLSSPAVITDKVIPACLPSPNYVVADRTECFITGWGETQGTFGAGLLKEAQLP
VIENKVCNRYEFLNGRVQSTELCAGHLAGGTDSCQGDSGGPLVCFEKDKYILQGVTSWGLGCARPNKPG
VYVRVSRFVTWIEGVMRNN
SEQ ID NO: 11 (nucleic acid sequence of Micro-plg (micro-plasminogen)): 
gccccttcatttgattgtgggaagcctcaagtggagccgaagaaatgtcctggaagggttgtagggggggtgtgtggcccacccacattcctggccctggcaagtcagtc
ttagaacaaggtttggaatgcacttctgtggaggcaccttgatatccccagagtgggtgttgactgctgcccactgcttggagaagtccccaaggccttcatcctacaagg
tcatcctgggtgcacaccaagaagtgaatctcgaaccgcatgttcaggaaatagaagtgtctaggctgttcttggagcccacacgaaaagatattgccttgctaaagctaa
gcagtcctgccgtcatcactgacaaagtaatcccagcttgtctgccatccccaaattatgtggtcgctgaccggaccgaatgtttcatcactggctggggagaaacccaa
ggtacttttggagctggccttctcaaggaagcccagctccctgtgattgagaataaagtgtgcaatcgctatgagtttctgaatggaagagtccaatccaccgaactctgtg
ctgggcatttggccggaggcactgacagttgccagggtgacagtggaggtcctctggtttgcttcgagaaggacaaatacattttacaaggagtcacttcttggggtcttg
gctgtgcacgccccaataagcctggtgtctatgttcgtgtttcaaggtttgttacttggattgagggagtgatgagaaataattaa
SEQ ID NO: 12 (amino acid sequence of Micro-plg (micro-plasminogen)): 
APSFDCGKPQVEPKKCPGRVVGGCVAHPHSWPWQVSLRTRFGMHFCGGTLISPEWVLTAAHCLEKSPRPS
SYKVILGAHQEVNLEPHVQEIEVSRLFLEPTRKDIALLKLSSPAVITDKVIPACLPSPNYVVADRTECFITGW
 
GETQGTFGAGLLKEAQLPVIENKVCNRYEFLNGRVQSTELCAGHLAGGTDSCQGDSGGPLVCFEKDKYIL
QGVTSWGLGCARPNKPGVYVRVSRFVTWIEGVMRNN
SEQ ID NO: 13 (nucleic acid sequence of the serine protease (structure) domain): 
gttgtagggggggtgtgtggcccacccacattcctggccctggcaagtcagtcttagaacaaggtttggaatgcacttctgtggaggcaccttgatatccccagagtgggt
gttgactgctgcccactgcttggagaagtccccaaggccttcatcctacaaggtcatcctgggtgcacaccaagaagtgaatctcgaaccgcatgttcaggaaatagaa
gtgtctaggctgttcttggagcccacacgaaaagatattgccttgctaaagctaagcagtcctgccgtcatcactgacaaagtaatcccagcttgtctgccatccccaaatt
atgtggtcgctgaccggaccgaatgtttc360
atcactggctggggagaaacccaaggtacttttggagctggccttctcaaggaagcccagctccctgtgattgagaataaagtgtgcaatcgctatgagtttctgaatgga
agagtccaatccaccgaactctgtgctgggcatttggccggaggcactgacagttgccagggtgacagtggaggtcctctggtttgcttcgagaaggacaaatacatttt
acaaggagtcacttcttggggtcttggctgtgcacgccccaataagcctggtgtctatgttcgtgtttcaaggtttgttacttggattgagggagtgatgaga
SEQ ID NO: 14 (amino acid sequence of the serine protease (structure) domain):

VVGGCVAHPHSWPWQVSLRTRFGMHFCGGTLISPEWVLTAAHCLEKSPRPSSYKVILGAHQEVNLEPHV
QEIEVSRLFLEPTRKDIALLKLSSPAVITDKVIPACLPSPNYVVADRTECFITGWGETQGTFGAGLLKEAQLP
VIENKVCNRYEFLNGRVQSTELCAGHLAGGTDSCQGDSGGPLVCFEKDKYILQGVTSWGLGCARPNKPG
VYVRVSRFVTWIEGVMR

## Claims

1. A method for promoting removal of a pathological protein by a ubiquitin proteasome system and an autophagy lysosome system, comprising administering to a subject an effective amount of one or more compounds selected from the group consisting of: a component of a plasminogen activation pathway, a compound capable of activating plasminogen directly or activating plasminogen indirectly by activating an upstream component of a plasminogen activation pathway, a compound mimicking the activity of plasminogen or plasmin, a compound capable of up-regulating the expression of plasminogen or a plasminogen activator, a plasminogen analog, a plasmin analog, a tPA or uPA analog, and an antagonist of a fibrinolysis inhibitor.

2. A method for removing different types of extracellular and/or intracytoplasmic and/or intranuclear pathological proteins, comprising administering to a subject an effective amount of one or more compounds selected from the group consisting of: a component of a plasminogen activation pathway, a compound capable of activating plasminogen directly or activating plasminogen indirectly by activating an upstream component of a plasminogen activation pathway, a compound mimicking the activity of plasminogen or plasmin, a compound capable of up-regulating the expression of plasminogen or a plasminogen activator, a plasminogen analog, a plasmin analog, a tPA or uPA analog, and an antagonist of a fibrinolysis inhibitor.

3. The method according to claim 1 or 2, wherein the component of the plasminogen activation pathway is selected from the group consisting of: plasminogen, recombinant human plasmin, Lys-plasminogen, Glu-plasminogen, plasmin, plasminogen and plasmin variants and analogs containing one or more kringle domains and protease domains of plasminogen and plasmin, mini-plasminogen, mini-plasmin, micro-plasminogen, micro-plasmin, delta-plasminogen, delta-plasmin, a plasminogen activator, tPA, and uPA.

4. The method according to claim 1 or 2, wherein the antagonist of the fibrinolysis inhibitor is an inhibitor of PAI-1, a complement C1 inhibitor, α2 antiplasmin or α2 macroglobulin, such as an antibody.

5. The method according to any one of claims 1 to 4, wherein the compound has one or more of the following activities: removing extracellular and/or intracytoplasmic and/or intranuclear pathological proteins, promoting the removal of pathological proteins by a ubiquitin proteasome system, promoting the removal of pathological proteins by an autophagy lysosome system, regulating to optimize the expression and/or activity of members of a ubiquitin system, regulating to optimize the expression and/or activity of LC3, regulating to optimize the expression and/or activity of members of an autophagy lysosome system, and regulating to optimize (especially promoting) the expression of LAMP2.

6. The method according to any one of claims 1 to 5, wherein the compound is plasminogen or plasmin.

7. The method according to any one of claims 1 to 6, wherein the plasminogen is Glu-plasminogen, Lys-plasminogen, or a conservatively substituted variant thereof.

8. The method according to any one of claims 1 to 7, wherein the plasminogen has at least 75%, 80%, 85%, 90%, 95%, 96%, 97%, 98% or 99% sequence identity with SEQ ID NO: 2 and has lysine binding activity and/or proteolytic activity of plasminogen.

9. The method according to any one of claims 1 to 8, wherein the plasminogen comprises one or more selected from the group consisting of:
1) a serine protease domain having the amino acid sequence represented by SEQ ID NO: 14;
2) a serine protease domain having at least 80%, 90%, 95%, 96%, 97%, 98%, or 99% identity with SEQ ID NO: 14 and retaining proteolytic activity;
3) a Kringle domain being one or more selected from the group consisting of Kringle 1, Kringle 2, Kringle 3, Kringle 4 and Kringle 5; and
4) a Kringle domain having at least 80%, 90%, 95%, 96%, 97%, 98%, or 99% identity with one or more selected from the group consisting of Kringle 1, Kringle 2, Kringle 3, Kringle 4 and Kringle 5, and retaining lysine binding activity.

10. The method according to any one of claims 1 to 9, wherein the plasminogen is selected from Glu-plasminogen, Lys-plasminogen, mini-plasminogen, micro-plasminogen, or delta-plasminogen, or a variant thereof retaining the proteolytic activity of plasminogen.

11. The method according to any one of claims 1 to 10, wherein the plasminogen comprises an amino acid sequence represented by SEQ ID NO: 2, 6, 8, 10 or 12, or comprises a conservatively substituted variant of the amino acid sequence represented by SEQ ID NO: 2, 6, 8, 10 or 12.

12. The method according to any one of claims 1 to 11, wherein the plasminogen is used in combination with one or more other treatment methods or drugs.

13. The method according to claim 12, wherein the other treatment method comprises cell therapy (comprising stem cell therapy), supportive therapy, and physical therapy.

14. The method according to any one of claims 1 to 13, wherein the plasminogen is administered by nasal inhalation, nebulized inhalation, nasal drops, eye drops, ear drops, intravenous, intraperitoneal, subcutaneous, sublingual, intracranial, intrathecal, intra-arterial (e.g., via carotid artery), or intramuscular administration.

15. The method according to any one of claims 1 to 14, wherein the pathological protein is one or more selected from the group consisting of: cystic fibrosis transmembrane conductance regulator (CFTR), α1-antitryspin, Parkin protein, p53 tumor suppressor protein, Wilms' Tumor 1 protein (WT1 protein), von Hippel Lindau (VHL) tumor suppressor protein, Merlin protein, Src protein kinase, Crystallin, Transthyretin, short-chain acyl-CoA dehydrogenase variant (SCAD variant), low density lipoprotein receptor, huntingtin, neurofilament protein, peripherin, 1α-intermediate filament protein (α-Internexin), islet amyloid polypeptide, β2-microglobulin , serum amyloid A protein , immunoglobulin light chains, human lysozyme, α-lactalbumin, prothymosin α, apolipoprotein E, apolipoprotein J, amyloid β-protein (Aβ), Tau protein, alpha-synuclein (α-syn), TAR DNA-binding protein (transactive response DNA-binding protein 43, TDP-43), prion protein, copper-zinc superoxide dismutase (SOD1), plasma ceruloplasmin, heavy chain IgG, light chain IgG, interferon-induced protein 6-16 (IF16-6, G1P3), mutated androgen receptor, and ataxin-3.
